# EUROPÄISCHE PATENTSCHRIFT

(11) **EP 2 101 867 B1**
(45) Veröffentlichungstag und Bekanntmachung des Hinweises auf die Patenterteilung: **17.04.2013**
(21) Anmeldenummer: 07856856.5
(22) Anmeldetag: 18.12.2007
(51) Int. Cl.: A61N 1/36

(54) **SEHHILFE MIT DREIDIMENSIONALER BILDERFASSUNG**
VISUAL AID WITH THREE-DIMENSIONAL IMAGE ACQUISITION
ASSISTANCE À LA VISION DOTÉE D'UNE SAISIE TRIDIMENSIONNELLE D'IMAGE

(30) Priorität: 19.12.2006 DE 102006060045
(43) Veröffentlichungstag der Anmeldung: 23.09.2009
(73) Patentinhaber: IMI Intelligent Medical Implants AG, 6304 Zug (CH)
(72) Erfinder: ZIEMECK, Patrick, 07548 Gera (DE); DAPPER, Marcus, 53227 Bonn (DE); KOPKA, Christian, 53175 Bonn (DE); HORNIG, Ralf, 53113 Bonn (DE)
(74) Vertreter: Graf von Stosch, Andreas
(86) Internationale Anmeldenummer: PCT/EP2007/011133
(87) Internationale Veröffentlichungsnummer: WO 2008/074478

(56) Entgegenhaltungen:
- WO-A-97/17043
- US-A- 5 106 179
- US-A1- 2002 101 568
- US-A1- 2004 117 011
- US-A1- 2004 236 389
- US-B1- 6 400 989

## Beschreibung

Die vorliegende Erfindung betrifft eine Sehhilfe mit dreidimensionaler Bild- und Informationserfassung. Eine Sehhilfe im Sinne der vorliegenden Erfindung umfasst auch andere Bilderfassungssysteme, wie z.B. Restlichtverstärker oder Nachtsichtgeräte zur Unterstützung des menschlichen Sehvermögens. Solche Sehhilfen bzw. Sehprothesen, wie z.B. ein Retina-Implantat, können auch Funktionen zur Bildervergrößerung oder zur Restlichtverstärkung umfassen.

Es wurden bereits Sehhilfen in Form von Implantaten für die Netzhaut (Retina) des menschlichen Auges entwickelt, die zur Behandlung von Patienten vorgesehen sind, deren Sehvermögen teilweise oder vollständig durch Defekte in der Retina verloren gegangen ist. Dabei wird eine mikroelektronische Vorrichtung im Bereich der Retina mit einer Vielzahl von lichtempfindlichen Pixelelementen implantiert, über die ein auf die Retina über den noch intakten natürlichen Strahlengang des Auges projiziertes Bild aufgenommen wird. Bei anderen Sehprothesen erfolgt die Bilderfassung durch eine externe Kamera, insbesondere eine Videokamera, die vorzugsweise in einer Brille untergebracht ist. Das durch die Pixelelemente bzw. die Kamera erfasste Bild wird in elektrische Signale umgesetzt und über Stimulations-Elektroden mittels elektrischer Stimulations-Impulse an die Ganglienzellen der Retina und den Sehnerv abgegeben, um so das Sehvermögen des Patienten wiederherzustellen bzw. zu verbessern. Die bekannten Sehhilfen haben jedoch den Nachteil, dass sie ausschließlich das über den natürlichen Strahlengang des Auges oder das von der externen Kamera erfasste Bild verarbeiten. Das dabei entstehende Bild ist deshalb lediglich zweidimensional und enthält keine dreidimensionale Informationen.

Um das natürliche Sehvermögen mittels einer Sehhilfe oder Sehprothese möglichst real wiederherzustellen bzw. zu unterstützen, ist es daher erstrebenswert, über die reine Erfassung eines zweidimensionalen Bildes hinaus zusätzliche Informationen in die Bildverarbeitung einzubeziehen.

Die Patentschrift DE10103922 bildet den Oberbegriff des Anspruchs 1. Es wird ein interaktives Datensicht- und Bediensystem mit einer in der Art einer Brille von einer Bedienperson tragbaren optischen Vorrichtung beschrieben. Es ist eine Signalverarbeitungseinrichtung vorgesehen, mit der die von der optischen Vorrichtung erfassten Signale auswertbar sind und ein entsprechendes Ausgangssignal an eine Kommunikationsschnittstelle übertragbar ist. Basierend auf diesem Ausgangssignal und zusätzlichen Steuerbefehlen kann die optische Vorrichtung zur Einspielung zusätzlicher Bildinformation auf die Netzhaut veranlasst werden.

Diese Aufgabe wird durch die erfindungsgemäße Vorrichtung mit den Merkmalen gemäß Anspruch 1 gelöst. Vorteilhafte Weiterbildungen der vorliegenden Erfindung sind in den Unteransprüchen gekennzeichnet.

Die vorliegende Erfindung löst die oben genannte Aufgabe durch ein Sehhilfe-System mit mindestens einer Kamera zum Erfassen eines Bildes mit einer Vielzahl von Bilddaten, elektronischen Mitteln zum Bearbeiten der erfassten Bilddaten und einer im Körper am Sehorgan implantierbaren Stimulations-Vorrichtung zur Wiedergabe des bearbeiteten Bildes durch entsprechende Stimulierung des Sehorgans, wobei bei der Wiedergabe des bearbeiteten Bildes zusätzliche Informationen und/oder bestimmter Attribute bezüglich der räumlichen Position von Objekten in dem erfassten Bild visualisiert werden, die mit Objekten in dem erfassten Bild assoziiert sind.

Mit einer Sehhilfe nach der vorliegenden Erfindung können insbesondere Objekte in der unmittelbaren Umgebung des Benutzers speziell visualisiert oder hervorgehoben werden, während weiter entfernte Objekte optional ausgeblendet werden können. Die Hervorhebung bestimmter Objekte kann auch durch Verwendung unterschiedlicher Graustufen oder verschiedener Farben erfolgen. So können beispielsweise in der näheren Umgebung befindliche Objekte in einer anderen Farbe oder Graustufe dargestellt werden als weiter entfernt liegende Objekte. Die vorliegende Erfindung bietet damit den Vorteil, dass ein dreidimensionales Abbild der aktuellen Umgebung vom Benutzer erfasst werden kann, indem das dem Benutzer von der erfindungsgemäßen Sehhilfe-System visualisierte Bild zusätzliche Informationen beispielsweise über den relativen Abstand der in dem Bild enthaltenen Objekte umfasst und damit eine tiefenselektive Darstellung des erfassten Bildes erreicht werden kann.

Der Vorteil der tiefen-selektiven Darstellung besteht darin, dass der Benutzer des Sehhilfe-Systems trotz einer begrenzten Pixelauflösung, Farbabstufung, Grauabstufung oder begrenzter Anzahl von Bildpunkten (Pixel) des Sehhilfe-Systems einen visuell gut strukturierten und leicht zu interpretierenden Bildeindruck der näheren oder weiter entfernten Umgebung erhalten kann. Die Umgebung des Benutzers kann dann beispielsweise über eine Umrissdarstellung visualisiert werden, was sich mit einer verhältnismäßig geringen Anzahl von gleichzeitig zu aktivierenden Bildpunkten realisieren lässt.

Nach der vorliegenden Erfindung umfasst das Gesamtsystem der Sehhilfe eine Brille und optional eine zusätzlich am Körper getragener Einheit, z.B. einem Pocketcomputer, der an beliebiger Stelle des Körpers getragen werden kann und über eine Drahtverbindung oder alternativ über eine drahtlose Funkverbindung mit der Brille kommunizieren und Bilddaten austauschen kann. Bei einer speziellen Anwendung der vorliegenden Erfindung auf ein oben beschriebenes Retina-Implantat-System kann zusätzlich eine im Körper implantierte Vorrichtung vorgesehen sein, welche die von der externen Kamera erfassten Bilddaten empfängt.

Bei einer bevorzugten Ausführungsform der vorliegenden Erfindung wird eine dreidimensionale Karte zur Aufbereitung des dargestellten Bildausschnitts verwendet. Eine dreidimensionale Karte der Umgebung eines bestimmten Bildausschnitts lässt sich in der Form verwenden, dass nur die Segmente des Bildes dargestellt werden, die in der näheren oder mittleren Entfernung des Benutzers der Sehhilfe vorhanden sind, da diese für den Benutzer von größerem Interesse sein können als Segmente des Bildes oder Objekte, die sich in einer größeren Entfernung befinden. Alternativ können in einem speziellen Betriebsmodus der erfindungsgemäßen Sehhilfe nur weiter entfernte Gegenstände dargestellt werden, falls der Benutzer der Sehhilfe vorzugsweise den Hintergrund des erfassten Bildes oder die weiter entfernten Objekte visualisieren will.

Die unterschiedlichen Betriebsmodi der Sehhilfe können entweder in der Bildverarbeitung des Systems fest eingebaut sein oder für den Benutzer optional wählbar zur Verfügung gestellt werden. Die Auswahl der Betriebsmodi kann über Bedienelemente der Brille, über den optional vorhandenen Pocketcomputer oder über eine Fernbedienung erfolgen, die an beliebiger Stelle des Körpers getragen werden kann.

Der Darstellungsbereich des Systems lässt sich durch entsprechend ausgewählte mathematischen Schnittflächen oder Oberflächenfunktionen in einem dreidimensionalen Modell des Pocketcomputers einstellen. Als Schnittflächen können beispielsweise Ausschnitte einer planaren Fläche oder einer Zylinder- oder einer Kugeloberfläche oder andere beliebig geformte Schnittflächen gewählt werden. Je nach Betriebsmodus des Implantat-Systems werden dadurch nur die Schnittflächen mit den erfassten Objekten in dem dreidimensionalen Modell des Pocketcomputers (oder der Brille) dargestellt.

Durch eine Einstellvorrichtung des Implantat-Systems kann der Benutzer der Sehhilfe einen solchen Betriebsmodus beispielsweise einen Scanmodus wählen, bei dem nur solche Objekte dargestellt werden, die sich in einem bestimmten Erfassungsbereich des Systems befinden. Ferner kann der Benutzer der Sehhilfe mittels der Einstellvorrichtung den optischen Erfassungsbereich des Systems variieren. In einem speziellen Betriebsmodus des erfindungsgemäßen Systems kann der optische Erfassungsbereich des Systems wiederholt erweitert und danach wieder kontrahiert werden, so dass der Benutzer der Sehhilfe kontinuierlich räumliche Schnittbilder der Umgebung präsentiert bekommt.

Gemäß einer weiteren bevorzugten Ausführungsform kann die erfindungsgemäße Sehhilfe mittels der elektronischen Rechenkapazität des Pocketcomputers anhand der erfassten Bilddaten eine dreidimensionale Karte erstellen und dem Benutzer visualisieren. Mit einer dreidimensionalen Karte kann dem Benutzer der Sehhilfe auch ein Bildausschnitt beispielsweise in einer Vogelperspektive präsentiert werden, in der ein Grundriss der aktuellen Umgebung und der darin befindlichen Objekte dargestellt wird, was insbesondere für die Navigation in unübersichtlicher Umgebung hilfreich sein kann.

Diese Betriebsmodi können nicht nur bei Sehhilfen für Benutzer mit stark verringertem Sehvermögen eingesetzt werden, sondern auch bei Visualisierungssystemen zur Orientierungshilfe für Benutzer mit normalem Sehvermögen. Die vorliegende Erfindung kann auch bei Sehhilfen angewendet werden, bei denen eine in der Brille integrierte Videokamera das Bild erfasst und über ebenfalls in der Brille integriertes Display auf die Netzhaut eines sehbehinderten Benutzers projiziert wird. Dabei können bereits grobe Pixeldarstellungen eine Verbesserung des Seheindrucks erzielen.

Bei einer weiteren bevorzugten Ausführungsform der erfindungsgemäßen Sehhilfe wird unter Verwendung von nur einer Kamera eine dreidimensionale Karte erzeugt. Dabei wird vorzugsweise die Kamera verwendet, die in der Brille des Benutzers zur Aufnahme der Bildinformation integriert ist, um eine stereoskopische Bildverarbeitung durchzuführen. Hierbei können Bilder von räumlich unterschiedlichen Positionen der Kamera genutzt werden, die mit geringem Zeitversatz nacheinander aufgezeichnet wurden. Dazu müssen die relativen räumlichen Positionen und die relativen Winkel-Orientierungen der Kamera berücksichtigt werden, was im folgenden Abschnitt erläutert wird.

Die Brille, in der die Kamera integriert ist, kann beispielsweise mit dreidimensionalen Beschleunigungs-Sensoren ausgestattet sein, die jeweils die Beschleunigung [*ẍ ÿ z̈*] der Brille und damit die Beschleunigung der Kamera in den drei Raumrichtungen [*x, y, z*] ermitteln. Alternativ kann die Brille, in der die Kamera integriert ist, mit einem sechsdimensionalen Beschleunigungs-Sensor ausgestattet sein, der jeweils sowohl die Beschleunigung der Brille und damit die Beschleunigung der Kamera in den drei Raumrichtungen [*x, y, z*] als auch die Winkelbeschleunigung [*ẍ ÿ z̈* ϕ̈*ₓ* ϕ̈*_{y}* ϕ̈*_{z}*] des Bezugssystems der Brille um die drei Raumachsen ermittelt. Die Dimensionalität eines Sensors gibt folglich die Anzahl der Messgrößen an, die ein Sensor ermitteln kann. Ein sechsdimensionaler Beschleunigungs-Sensor ermittelt beispielsweise drei translatorische Beschleunigungen und drei Winkelbeschleunigungen, die jeweils in einem orthogonalen Bezug zueinander stehen, und ermittelt damit insgesamt 6 orthogonale Beschleunigungsmesswerte.

Um die aktuelle Kameraposition relativ zu einer vorhergehenden Kamerapositionen zu bestimmen, kann ein Trägheitsnavigationsverfahren verwendet werden. Dazu können ein, zwei oder mehr Beschleunigungs-Sensoren (eindimensionale, zweidimensionale, dreidimensionale oder mehr-dimensionale, z.B. sechsdimensionale Beschleunigungs-Sensoren in der Brille an kinematisch gut konditionierten Positionen platziert werden. Kinematisch gut konditionierte Positionen sind beispielsweise solche Positionen, die innerhalb der Brille hinreichend weit voneinander entfernt sind.

Durch zweifache Integration der oben genannten sechs orthogonalen Beschleunigungsmesswerte kann die relative Position [Δ*x* Δ*y* Δ*z* Δϕ*ₓ* Δϕ*_{y}* Δϕ*_{z}*] der Brille bzw. die Kameraposition relativ zu einer vorhergehenden Kamerapositionen bestimmt werden. Die Integration der Beschleunigungsmesswerte und die Berechnung der Position und Orientierung der Brille erfolgt vorteilhaft durch elektronische Integratoren in einer in dem Sehhilfe-System integrierten Recheneinheit, die entweder in der Brille oder außerhalb der Brille, z.B. in einer am Körper des Benutzers getragenen Einheit ("Pocketcomputer"), untergebracht ist oder auf die Brille und die am Körper getragenen Einheit verteilt sein kann. Dabei kann die am Körper getragene Einheit entweder per Drahtverbindung und oder über Funkverbindung (z.B. mittels Blue Tooth, WLAN oder andere Kommunikationstechniken) mit der Brille kommunizieren.

Die zeitliche Intergrationsschrittweite bei der Integration der Beschleunigungsmesswerte sollte hinreichend kleiner sein als die Zeitspanne, innerhalb der sich die Beschleunigung der Brille wesentlich ändert, um über einen begrenzten Zeitbereich, z.B. 10 Sekunden, hinreichend genaue Positionsänderungen der Brille bzw. Kamera messen zu können, mit denen dann mindestens zwei oder mehr Kamerabilder aus unterschiedlichen Positionen der Kamera erfasst werden können.

Mittels der oben genannten zweifachen Integration wird aus den sechs orthogonalen Beschleunigungsmesswerten eines 6D-Beschleunigungsvektors ein sechsdimensionaler relativer Positionsvektor generiert, der die Differenz einer vorhergehenden Position der Kamera zu einer nachfolgenden Kameraposition angibt. Dieser sechsdimensionale relative Positionsvektor enthält drei Ortsdifferenzkoordinaten (Δx, Δy, Δz), welche die Differenz der Ortskoordinaten (x, y, z) der vorhergehenden Kameraposition zur nachfolgenden Kameraposition angeben, und drei Winkeldifferenzkoordinaten (Δϕ*ₓ*, Δϕ*_{y}*, Δϕ*_{z}*), welche die Differenz der Winkelposition (ϕₓ, ϕ_{y}, ϕ_{z}) der vorhergehenden Kameraposition zur nachfolgenden Kameraposition angeben. Die Integration kann in diskreten Zeitschritten erfolgen oder kontinuierlich durchgeführt werden.

Auf diese Weise kann eine Sequenz von Kamerabildern bei jeweils unterschiedlichen Kamerapositionen erfasst werden, wobei eine Sequenz aus mindestens zwei Kamerabildern besteht. Jedem Kamerabild dieser Sequenz ist eine relative Position in Bezug auf das vorangehende oder das nachfolgende Kamerabild zugeordnet, beispielsweise mittels der oben genannten Ortsdifferenzkoordinaten und Winkeldifferenzkoordinaten. Es können auch Kamerabilder in festen Zeitintervallen mit korrelierter Position aufgenommen werden, wobei ältere Bilder optional in ihrem Einfluss auf die aktuelle Raumberechnung zunehmend schwächer gewichtet werden können, bis sie schließlich keinen Einfluss mehr haben. Auf diese Weise wird dem Effekt entgegengewirkt, dass die Positionsbestimmung mittels der Trägheitsnavigation über größere Zeitspannen zunehmende Ungenauigkeiten in Form einer Positionsdrift aufweisen kann.

Bei einer weiteren bevorzugten Ausführungsform der erfindungsgemäßen Sehhilfe kann eine Zuordnung von Kamerabildern und Kamerapositionen zu verschiedenen Messzeitpunkten vorgenommen werden. Dadurch kann eine zweite Kamera, die ansonsten zur Aufnahme von Stereobildern benötigt würde, vorteilhaft eingespart werden, ohne auf dreidimensionale Bilder zu verzichten. Ferner können mehr als zwei Bilder aus unterschiedlichen Positionen und Orientierungen verarbeitet werden, um dadurch eine vollständigere dreidimensionale Karte der Umgebung des Benutzers der Sehhilfe zu erzeugen, was z.B. mittels des bekannten "Bündelausgleichsverfahren" geschehen kann. Eine Voraussetzung für dieses Verfahren sind leichte Kopfbewegungen des Benutzers in der Größenordnung des Augenabstandes zweier menschlicher Augen. Die Kamera kann dann jeweils ein Bild aus unterschiedlichen Positionen erfassen und damit in kurzer zeitlicher Abfolge die beiden Bilder der Recheneinheit liefern, die ein Mensch mit normalem Sehvermögen mit beiden Augen erfassen würde. Die Recheneinheit kann daraufhin die beiden Kamerabilder getrennt aufbereiten und das dreidimensionale Modell im Pocketcomputer aktualisieren und erweitern.

Darüber hinaus kann zu Beginn der oben genannten Integration eine Messung des Geschwindigkeits-Offsets durchgeführt werden, um die Messgenauigkeit und damit die Zuordnung der erfassten Kamerabilder zu den berechneten Orts- und Winkelpositionen der Kamera zu verbessern. Dies kann auf verschiedene Möglichkeiten erfolgen:

Der Benutzer der Sehhilfe kann durch entsprechende Eingabemittel die Geschwindigkeit der Brille auf Null setzen, z.B. beim Stillhalten der Brille für eine kurze Zeitspanne. Dies kann zum Beispiel auch durch kurzes Ablegen der Brille auf eine unbewegte Unterlage erfolgen. Bei hinreichend hoher Genauigkeit der Beschleunigungssensoren und der elektronischen Integratoren kann es auch ausreichen, die Brille während des Ladevorganges, wo sie sich mit hoher Wahrscheinlichkeit in Ruhe befindet, mit dem Geschwindigkeits-Offset Null zu referenzieren und von diesem Zeitpunkt an die Integration zu starten und diesen Geschwindigkeits-Offset für die gesamte tägliche Benutzung zu verwenden.

Die aktuelle Absolut-Geschwindigkeit kann auch mit Hilfe anderer Navigationshilfsmittel beispielsweise mittels GPS oder vergleichbarer Funk-Triangulationsverfahren bestimmt werden. Ferner kann die aktuelle Geschwindigkeit der Brille bzw. des Benutzers der Sehhilfe über die Veränderung der Bildinformation in Kombination mit den Beschleunigungsmessdaten der Brille ermittelt werden. Neben der oben erwähnten Geschwindigkeitsermittlung besteht auch die Möglichkeit, die aktuelle Position der Brille des Benutzers mittels GPS oder vergleichbarer Funk-Triangulationsverfahren zu bestimmen. Die Orientierung der Brille im Raum, d.h. die Winkelpositionen der Brille kann durch Auswertung der vergangenen Winkelpositionswerte ermittelt werden oder z.B. durch einen Kompass (z.B. einer Hallsonde) oder durch vergleichbare Orientierungs-Messverfahren. Der Winkel um die Nick-Achse der Brille (Neigungswinkel), der durch einen Kompass nicht erfassbar ist, kann beispielsweise durch einen gravitationsbasierten Neigungssensor ermittelt werden.

Es kann auch eine Kombination der oben genannten Verfahren eingesetzt werden, um den Messfehler der in der Brille integrierten Sensoren zu minimieren, insbesondere um Ausnahmen, die bei den verschiedenen Verfahren auftreten können, spezifisch zu behandeln. Dies gilt insbesondere für den Fall, dass sich Teile der Umgebung des Benutzers schnell bewegen oder der Benutzer sich in einem schnell bewegten Inertialsystem, wie z.B. einem Zug, Auto, oder Flugzeug befindet.

Gemäß einer weiteren bevorzugten Ausführungsform der erfindungsgemäßen Sehhilfe erfolgt die Gewinnung stereoskopischer bzw. dreidimensionaler Informationen bezüglich der räumlichen Umgebung im Blickfeld des Benutzers durch die Verwendung einer zweiten Kamera in der Brille. Dabei ist die zweite Kamera vorzugsweise ebenfalls in der Brille integriert und möglichst weit von der ersten Kamera in der Brille entfernt. Aufgrund der bekannten, festen Entfernung der beiden Kameras voneinander lassen sich aus den beiden, leicht unterschiedlichen Bildern, stereoskopische Tiefeninformationen über Segmente des aktuellen Bildausschnitts berechnen, die von der elektronischen Recheneinheit zu einer dreidimensionalen Karte der Umgebung des Benutzers umgerechnet und über Stimulationsmittel dem Benutzer vermittelt werden können.

Bei noch einer weiteren bevorzugten Ausführungsform der erfindungsgemäßen Sehhilfe werden alternativ oder zusätzlich Abstandssensoren verwendet, wie z.B. Ultraschallsensoren, rotierende Laserscanner im sichtbaren oder unsichtbaren Wellenlängenbereich, z.B. im Ultraviolett- oder Infrarot-Lichtbereich. Mit Hilfe solcher Abstandssensoren kann die Umgebung des Implantatträgers besser erfasst und damit auch Hindernisse besser erkannt werden. Die von den Abstandssensoren erfassten Informationen können beispielsweise per Triangulationsverfahren weiterverarbeitet und entsprechenden Ausschnitten des erfassten Kamerabildes oder der Kamerabild-Sequenzen zugeordnet werden. Dabei können z.B. auch mehrere Sensoren in Form eines eindimensionalen oder mehrdimensionalen Arrays angeordnet werden. Bei dieser bevorzugten Ausführungsform der vorliegenden Erfindung umfasst die Brille ein- oder mehrdimensionale Arrays aus Sensoren oder Aktoren, wie z.B. Ultraschallsensoren, Infrarot-Sensoren, Photosensoren, Lasern oder anderen Sensoren oder Aktoren oder Kombinationen daraus.

Bei einer weiteren bevorzugten Ausführungsform der erfindungsgemäßen Sehhilfe wird eine Streifenprojektion oder strukturierte Beleuchtung vorgenommen. Dabei wird ein homogenes oder ein strukturiertes (z.B. "Gray kodiertes") Streifenmuster auf die Umgebung des Benutzers projiziert, vorzugsweise mit infrarotem Licht, z.B. mit Laserlicht. Die Erzeugung eines Streifenmusters erfolgt beispielsweise durch eine Projektionsmaske, die vor einem Lichtstrahl positioniert wird. Hierbei können alternativ z.B. bei Verwendung eines Laserstrahls Interferenz-Effekte vorteilhaft genutzt werden, um in der Umgebung des Benutzers ein entsprechendes Muster zu generieren. Alternativ kann z.B. ein Lichtstrahl mit Hilfe eines Mikrospiegelsystems abgelenkt werden und parallel zeitlich moduliert werden, um das aktuelle Blickfeld des Benutzers zu erfassen.

Das Streifenmuster ist bei Verwendung von Infrarot-Licht unsichtbar. Die Kamera in der Brille der Sehhilfe ist jedoch sensitiv für Infrarot-Licht und kann das Streifenmuster erfassen. Verzerrungen dieses Streifenmusters (z.B. Verbiegungen, Verschiebungen) können durch entsprechende Triangulationsverfahren in dreidimensionale Tiefeninformationen zurück transformiert werden, wodurch sich wiederum eine dreidimensionale Karte des aktuellen Bildausschnitts berechnen lässt.

Hierbei kann das Streifenmuster auch periodisch in definierten Zeitabschnitten aktiviert werden, die zwischen den Zeitpunkten liegen, zu denen das Original-Videobild aufgenommen wird (z.B. 10ms Streifenmuster, 490ms kein Streifenmuster). Hierdurch ist gleichzeitig auch das tatsächlich Abbild der Umgebung verfügbar, was zum Beispiel für ein sogenantes "Texture Mapping" der aktuellen Umgebung auf die aktuell dargestellten Objekte angewendet werden kann. Statt eines "Texture Mapping" können mit der dreidimensionalen Karte auch die aktuell nicht dargestellten Objekte ausmaskiert werden, so dass nur die Bildausschnitte der aktuell dargestellten Objekte sichtbar werden, was mit verhältnismäßig geringem Berechnungsaufwand durchführbar ist. Auf diese Weise kann die Umgebung des Benutzers der Sehhilfe übersichtlicher dargestellt werden.

Bei einer weiteren bevorzugten Ausführungsform der erfindungsgemäßen Sehhilfe ist die Brille mit einer Kamera und einem Gitterprojektor, wie z.B. einem Laserprojektor mit Ablenkeinheit, mit einer Projektionsmaske mit Lichtquelle oder anderen Projektionsmitteln ausgestattet. Bei dieser Ausführungsform erfolgt die Abtastung des vom Sehhilfe-System erfassten Sichtfeldes mittels eines Laserstrahls, der mit einer entsprechenden Ablenkeinheit im Strahlengang des Lasers positioniert wird, wie z.B. einem piezotechnisch angetriebenen Mikrospiegelsystem oder Drehspiegelsystem. Dabei kann das Kamerabild zur Positionsbestimmung des Bildpunktes genutzt werden, woraus sich dann z.B. durch Triangulation wieder die Position des reflektierenden Objektsegmentes ermitteln lässt. Alternativ kann auch ein schneller XY-Photodetektor eingesetzt werden, der die aktuelle Position des Bildpunktes in der Umgebung des Benutzers ermitteln kann und so eine Abtastung der gesamten Benutzerumgebung im Sekundenbereich ermöglicht.

Bei einer weiteren bevorzugten Ausführungsform der erfindungsgemäßen Sehhilfe wird eine Laufzeitmessung des reflektierten Laserlichtes mit Hilfe eines schnellen Photodetektors vorgenommen. Hierbei wird die Laufzeit zwischen dem Sendezeitpunkt des jeweiligen Laserlicht-Impulses und dem Empfangszeitpunkt des reflektierten Laserlicht-Impulses in dem Photodetektor ermittelt und daraus unter Berücksichtigung der Lichtgeschwindigkeit die Entfernung des betreffenden Bildpunktes berechnet. Anschließend lässt sich durch Auswertung der Messwerte für das Bildraster eine dreidimensionale Karte des betreffenden Bildausschnitts erstellen.

Gemäß einer weiteren bevorzugten Ausführungsform der vorliegenden Erfindung kann die von dem Pocketcomputer erzeugte dreidimensionalen Karte auch in einem externen dreidimensionalen Datenspeicher abgelegt werden, wobei besondere Objekte, kritische Geländeformen oder wichtige Gegenstände in dem vom Sehhilfe-System erfassten Bildbereich speziell gekennzeichnet oder hervorgehoben werden. So können beispielsweise folgende besondere Objekte einer alltäglichen Umgebung in der dreidimensionalen Karte des Sehhilfe-Systems besonders gekennzeichnet werden: Treppenstufen, Laternenpfähle, Ampeln, Zebrastreifen, Bordsteinkanten, Bodenunebenheiten, Kraftfahrzeuge, Fahrräder oder andere Fahrzeuge verschiedener Arten und Formen, Personen, Gesichter, Körperteile, Türrahmen, Fensterrahmen, Tische, Geschirr oder andere wichtige Gegenstände, die der besonderen Aufmerksamkeit des Betrachters bzw. des Benutzers des Sehhilfe-Systems bedürfen.

Die besonderen Objekte werden vorzugsweise automatisch durch Vergleich mit einer im Pocketcomputer oder im externen Datenspeicher abgelegten Bild-Bibliothek detektiert. Diese Bild-Bibliothek kann mit beliebigen Bild-Mustern gefüllt werden, die für den Benutzer des Sehhilfe-Systems von Bedeutung sein könnten, wie z.B. Aufnahmen einer Ampel oder von anderen wichtigen Gegenständen aus verschiedenen Blickrichtungen. Durch permanenten Vergleich der vom Sehhilfe-System erfassten oder aufgezeichneten Videobilder mit den in der Bild-Bibliothek gespeicherten Bildmuster können diese besonderen Objekte im Videobild automatisch erkannt und anschließend in der dreidimensionalen Karte gekennzeichnet werden.

Die Erfassung von Bildbestandteilen in der vom Pocketcomputer erzeugten dreidimensionalen Karte kann beispielsweise durch die bekannte Gabor-Filterung oder andere Verfahren der Bildsegmentierung erfolgen. Dazu kann z.B. jeder Bildpunktvektor Pi = (xᵢ, yᵢ, zᵢ) der dreidimensionalen Karte mit einer Eigenschaftsvariable Eᵢ versehen werden, woraus sich ein erweiterter Bildpunktvektor Pᵢ = (xᵢ, yᵢ, zᵢ, Eᵢ) mit (i = 1,...,N) ergibt. Diese Eigenschaftsvariable Eᵢ kann entweder auf binäre Weise einfache Attribute repräsentieren, wie z.B. wichtig oder unwichtig, oder auch eine Einteilung in verschiedene Klassen darstellen, wie z.B. uninteressant, interessant, sehr interessant, hilfreich oder gefährlich. Objekte mit einer dieser Attribute bzw. Eigenschaften können dann in dem vom Sehhilfe-System dargestellten Bildausschnitt mittels spezieller Effekte visualisiert werden, z.B. durch Blinken, einer Darstellung mit hoher Helligkeit, durch spezielle Farbgebung oder andere Markierungen.

Gemäß einer weiteren bevorzugten Ausführungsform des erfindungsgemäßen Sehhilfe-Systems erfolgt die Abstandsvermessung von Bildpunkten bzw. Gegenständen in der Umgebung des Benutzers mit Hilfe eines Laser-Abstandssensors. Ein solcher Laser-Abstandssensor umfasst üblicherweise einen um die z-Raumachse rotierenden Laserstrahl, der bei jeder Umdrehung die Umgebung zeilenweise abtasten kann, wobei je nach Montageposition eine Abtastung bis zu einem vollständigen Winkelumfang von 360° möglich ist. Die Laserstrahlquelle ist auf einer rotierenden Plattform positioniert, die ihrerseits den aktuellen Drehwinkel z der Plattform (0° bis 360°) erfassen kann.

Die Laserquelle eines Laser-Abstandssensors sendet in zeitlich hinreichend weit voneinander entfernten Intervallen Laserlicht-Impulse, die an Objekten in der abgetasteten Umgebung reflektiert werden und deren Wieder-Eintreffen am Ort ihrer Aussendung mittels eines Photodetektors zeitlich gemessen wird. Die Erfassung des ausgesendeten und reflektierten Lichtimpulses wird beispielsweise mit Hilfe einer Photodiode durchgeführt und die Laufzeit des Lichtimpulses mit einer hinreichend schnellen Elektronik ermittelt. Aus dem gemessenen Zeitintervall (Laufzeit t) zwischen dem Aussenden des Laserimpulses und dem Wiedereintreffen des Laserimpulses und der Lichtgeschwindigkeit (c = 3 10⁸ m/s) lässt sich die Entfernung s des von dem Laserpuls getroffenen Objektes aus dem Produkt nach der Formel s = ½ v t berechnen. Ein Vorteil der Abstandsvermessung mittels eines Laser-Abstandssensors besteht darin, dass durch den Laserstrahl ein sehr kleiner Bildpunkt auf die Umgebung projiziert werden kann, sodass sich eine detaillierte Abtastung und Auflösung der Objektoberfläche erzielen lässt.

Um ein vollständiges Bild von der Umgebung zu erhalten und die gesamte Bildoberfläche zu erfassen, ist es notwendig, mehr als nur eine Bildzeile abzutasten. Dazu muss der Laserstrahl des Laser-Abstandssensors zusätzlich in vertikaler Richtung abgelenkt werden, um die Bildspalten in einem gewünschten Winkelausschnitt abzutasten. Dies kann zum Beispiel mit einem um die x- Achse schwenkbaren Spiegel geschehen, der gleichfalls mit einer Winkelmess-Vorrichtung für den Winkel x um die x-Raumachse ausgestattet ist. Anhand der Winkel *x*, *z* und der Entfernung s lässt sich per Triangulation direkt die Position Ps = (xₛ, yₛ, zₛ) des anvisierten Bildpunktes relativ zu der Laserlichtquelle berechnen und als Raumpunkt in die vom Sehhilfe-System erzeugte dreidimensionale Karte übernehmen.

Als Laserquelle lässt sich zum Beispiel ein sogenannter VCSEL (Vertical Cavity Surface Emitting Laser) verwenden. Ein solches Bauteil weist eine geringe Kantenlänge von ca. 300µm auf und lässt sich daher gut in der Brille des Sehhilfe-Systems integrieren. Als vertikale Ablenkeinheit kann z.B. ein handelsüblicher Mikrospiegel verwendet werden, wie er z.B. bereits in Video-Beamern zum Einsatz kommt. Die Rotation des Mikrospiegels um die z-Raumachse kann beispielsweise mit einem rotierenden, verspiegelten Prisma erfolgen, das durch einen Mikromotor angetrieben wird. Dadurch können Abmessungen für die gesamte Baueinheit unterhalb von 10mm Kantenlänge erzielt werden, die in der Brille des Sehhilfe-Systems untergebracht werden können. Wenn ein VCSEL mit Infrarot-Laserlicht außerhalb des sichtbaren Spektralbereichs verwendet wird, so kann die Abtastung der Umgebung erfolgen, ohne dass dabei Personen in der Umgebung des Benutzers gestört werden oder davon Notiz nehmen können.

Wie oben beschrieben, kann die dreidimensionale Informationserfassung und weitere Verarbeitung der erfassten Bilddaten des Sehhilfe-Systems durch eine extern generierte dreidimensionale Karte der erfassten Bilddaten unterstützt werden. Die dreidimensionale Karte wird vorzugsweise mittels der elektronischen Komponenten des Pocketcomputers generiert und auf dem Pocketcomputer oder auf einem drahtlos (z.B. per WLAN oder Bluetooth) mit dem Sehhilfe-System in Verbindung stehenden externen dreidimensionalen Datenspeicher, wie z.B. einem elektronischer Server, gespeichert.

Die dreidimensionale Karte kann auch als elektronisches Kartenmaterial über externe Quellen zur Verfügung gestellt werden, beispielsweise durch eine Karte für Navigationssysteme, die über eine CD oder andere Speichermedien oder per Internetanschluss auf den Server oder den Pocketcomputer übertragen werden. Bei einem solchen elektronischen Kartenmaterial ist in der Regel die Umgebung in Form von Gitternetzen abgebildet, auf die optional ein per Video aufgezeichnetes Bild der Welt-Oberfläche aufgewickelt bzw. projiziert werden kann.

Bei der dreidimensionalen Karte kann es sich auch um speziell für ein Retina-Implantat-System angefertigte oder aufbereitete dreidimensionale Informationen handeln, die eine detaillierte Form der näheren Umgebung enthalten. Die hinreichende Minimal-Auflösung einer solchen Darstellung liegt vorzugsweise in der Größenordnung von wenigen Millimetern bis Zentimetern. Die dreidimensionale Karte kann beispielsweise N einzelne Gitterpunkte Pᵢ = (xᵢ, yᵢ, zᵢ) mit (i = 1,...,N) enthalten, die mit bestimmten Bildpunkten bzw. Oberflächenpunkten der betreffenden Umgebung korrespondieren. Diese Gitterpunkte können durch Linien mit benachbarten Punkten verbunden werden, um so ein Gitternetz aufzuspannen.

Auf die in diesem Gitternetz in der dreidimensionalen Karte aufgespannten Flächen kann ein elektronisch aufgezeichnetes Bild beispielsweise in Form eines Fotos der Umgebung aufgewickelt bzw. projiziert werden. Dies erfolgt vorteilhaft mit dem bekannten Verfahren des sogenannten "Texturemapping", wodurch eine dreidimensionale Abbildung der Umgebung entsteht. Anhand der Position P_{B} = (x_{B}, y_{B}, z_{B}) und Orientierung B = (Bₓ, B_{y}, B_{z}) des Betrachters bzw. der Brille des Benutzers des Sehhilfe-Systems kann dann das aktuell sichtbare Blickfeld des Benutzers mit Hilfe von bekannten Standard Projektions-, Skalierungs- und Rotationsverfahren aus der Matrix-Algebra berechnet werden. Solche Verfahren sind bereits unter der Bezeichnung "Ray-Tracing" (Strahl-Rückverfolgung), "Vektorgrafik" bzw. "Texturemapping" (Bildprojektion auf dreidimensionalen Gitternetzkörper) bekannt.

Die Position P_{B} und Orientierung B des Betrachters bzw. der Brille wird mit Hilfe des Pocketcomputers ermittelt, um den aktuell erfassten Bildausschnitt darzustellen. Dies erfolgt mit dem oben beschriebenen Trägheitsnavigationsverfahren oder z.B. per GPS-Navigation. Es kann auch ein präziseres, lokal referenziertes, GPS-ähnliches Navigationsverfahren verwendet werden, das per Funktriangulation durch Messung von Laufzeitunterschieden mittels einer Anzahl M von Referenzsendem, die Position P_{B} des Betrachters hinreichend genau im Bereich von Millimetern oder Zentimetern bestimmen kann. Die exakte Absolutposition P_{Rj} = (X_{Rj}, Y_{Rj}, Z_{Rj}), mit (j = 1,..., M) der Referenzsender ist dabei vorzugsweise in dem Pocketcomputer gespeichert.

Die räumliche Ausrichtung bzw. die Orientierung B der Brille des erfindungsgemäßen Sehhilfe-Systems kann auch über einen gravitationsbasierten Neigungssensor oder andere Verfahren (z.B. Funktriangulation) bestimmt werden. Das erfasste Videobild der Kamera in der Brille kann mit dem dreidimensionalen Gitternetzmodell optional kombiniert werden und z.B. für das "Texturemapping" genutzt werden. So kann der möglicherweise störende Hintergrund des Videobildes ausgeblendet werden, um nur die in der Nähe des Benutzers befindlichen Objekte darzustellen. Alle störenden Hintergrundteile des Bildes können so z.B. schwarz dargestellt werden d.h. im Implantat findet an diesen Stellen keine elektrische Stimulation statt. Dabei liefern die im Vordergrund liegenden Objekte des dreidimensionalen Gittemetzmodells mit ihrem Umriss die Begrenzungslinien zu den Ausschnitten des Videobildes, die dargestellt werden sollen.

Die Generierung der dreidimensionalen Karte für die Umgebung des Benutzers kann auch mittels elektronischer Komponenten durchgeführt werden, die nicht in der Brille des Sehhilfe-Systems integriert sind. Die von der Brille erfassten Videobilder und die jeweils korrespondierende Position P_{B} und Orientierung B der Brille können dann oder per Funk oder eine Kabelverbindung an den Pocketcomputer oder an einen dreidimensionalen Server übertragen werden, der anhand dieser Daten die dreidimensionale Karte der Umgebung generieren bzw. aktualisieren kann. Dazu kann beispielsweise das sogenannte Bündelausgleichsverfahren oder andere Verfahren eingesetzt werden.

Für das oben genannte Trägheitsnavigation kommen prinzipiell Beschleunigungssensoren und elektronische Integratoren zu Anwendung, wobei die Messwerte der Beschleunigungssensoren mittels der elektronischen Integratoren integriert werden. Ein Beschleunigungssensor besteht in der Regel aus einer beschleunigten Referenzmasse m, die in einem Träger federnd gelagert ist. Die Position der Masse relativ zu dem Träger kann mit kammähnlichen Messstrukturen kapazitiv bestimmt werden. Dazu sind die kammähnlichen Messstrukturen sowohl am Träger als auch an der Referenzmasse befestigt. In Abhängigkeit von der Strecke s, um die sich die ineinander greifenden kammähnlichen Messstrukturen gegeneinander verschieben, ergibt sich eine Änderung der elektrischen Kapazität der Anordnung und damit ein entsprechendes Maß für die mechanische Beschleunigung a. Mit bekannter Federhärte D der Lagerung der Referenzmasse ergibt sich die Federkraft aus der Formel F = D · s und durch gleichsetzen der bekannten Formel für die Beschleunigungskraft F = m · a ergibt sich das Maß für die Beschleunigung a der Referenzmasse mit a = (D/m) · s. Solche in Halbleiter-Prozessen hergestellten Silizium-Beschleunigungssensoren mit geringen Abmessungen sind bereits in dreidimensionaler Ausführung erhältlich, wie z.B. der Baustein "ADXL 330" der Firma Analog Devices.

Beim Verfahren der Trägheitsnavigation ist es von Vorteil, den Beschleunigungssensor um alle drei Raumachsen drehbar in einem Gyroskop zu lagern, um eine Kreiselstabilisierung, d.h. eine definiert fixierte Raum-Ausrichtung der Anordnung zu erreichen. Dadurch kann die Anzahl der notwendigen Beschleunigungssensoren auf einen einzigen dreidimensionalen xyz-Sensor reduziert werden.

Ein elektronischer Integrator kann entweder analog aufgebaut werden mit Hilfe von präzisen Operations-Verstärkerschaltungen oder bei Einsatz eines Digitalrechners mit diversen mathematischen Integrationsverfahren realisiert werden, die ihre Präzision vorzugsweise dem Eingangssignal anpassen. Einige bekannte Integrationsverfahren sind zum Beispiel das Euler-Verfahren, das Runge-Kutta-Verfahren, das Bulirsch-Stoer-Verfahren und das Adams-Gear-Verfahren. Zur Realisierung eines elektronischen Integrators können auch Verfahren angewendet werden, die auf Abwandlungen oder Kombinationen der oben genannten Verfahren basieren. Ein numerisches Integrationsverfahren ist in der Regel präziser als elektrische Integrationsverfahren und wird daher bei dem erfindungsgemäßen Sehhilfe-System vorzugsweise eingesetzt.

Im Folgenden wird die vorliegende Erfindung anhand von bevorzugten Ausführungsbeispielen unter Bezugnahme auf die beigefügten Zeichnungen näher erläutert. Es zeigen:
- Figur 1: eine schematische Darstellung des Sehhilfe-Systems gemäß einer bevorzugten Ausführungsform der vorliegenden Erfindung mit einer drahtgebundenen Kopplung zwischen der Brille und dem Pocketcomputer- Modell;
- Figur 2: eine schematische Darstellung des Sehhilfe-Systems gemäß einer weiteren bevorzugten Ausführungsform der vorliegenden Erfindung mit einer drahtlosen Kopplung zwischen der Brille und dem Pocketcomputer;
- Figur 3: eine schematische Darstellung des Sehhilfe-Systems gemäß einer weiteren bevorzugten Ausführungsform der vorliegenden Erfindung mit dreidimensionalen oder n-dimensionalen Beschleunigungs-Sensoren, wobei n = 1, 2, 3, 4, 5, 6, ...;
- Figur 4: eine schematische Darstellung des Sehhilfe-Systems gemäß einer weiteren bevorzugten Ausführungsform der vorliegenden Erfindung mit einem sechsdimensionalen Beschleunigungs-Sensor;
- Figur 5: eine schematische Darstellung der in dem erfindungsgemäßen Sehhilfe-System durchgeführten Berechnung zur Positionsbestimmung des Sehhilfe-Systems gemäß einer bevorzugten Ausführungsform der vorliegenden Erfindung;
- Figur 6: eine schematische Darstellung einer Bewegungsspur mit einer Anzahl von unterschiedlichen aufeinander folgenden Positionen des Sehhilfe-Systems nach der vorliegenden Erfindung;
- Figur 7: eine schematische Darstellung des Sehhilfe-Systems gemäß einer weiteren bevorzugten Ausführungsform der vorliegenden Erfindung mit zwei Kameras zur Erfassung eines stereoskopischen Bildes;
- Figur 8: eine schematische Darstellung des Sehhilfe-Systems gemäß einer weiteren bevorzugten Ausführungsform der vorliegenden Erfindung mit Sensor-Arrays; und
- Figur 9: eine schematische Darstellung des Sehhilfe-Systems gemäß einer weiteren bevorzugten Ausführungsform der vorliegenden Erfindung mit einem Gitter-Projektor.

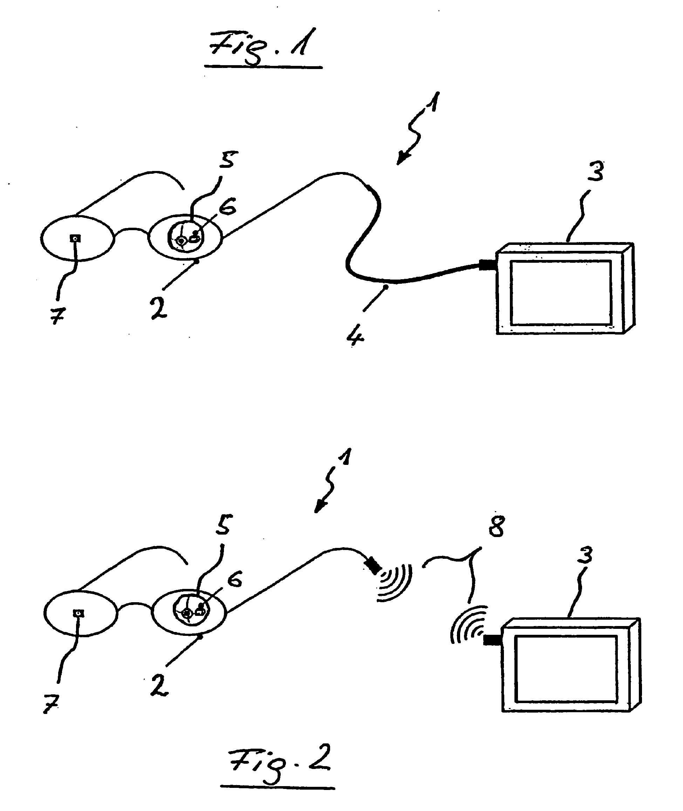

In Figur 1 ist eine schematische Darstellung des Sehhilfe-Systems 1 gemäß einer bevorzugten Ausführungsform der vorliegenden Erfindung gezeigt. In dieser Ausführungsform umfasst das erfindungsgemäße Sehhilfe-System 1 eine Brille 2, die in ihrer Form einer normalen Brille ähnelt und vom Benutzer des Sehhilfe-Systems 1 auch wie eine normale Brille getragen werden kann. In der Brille 2 ist eine elektronische Kamera 7 integriert, die Videobilder im Sichtbereich des Sehhilfe-Systems erfasst und in elektrische Bildinformationen mit Bildpunkten (Pixel) transformiert. Bei der in Figur 1 dargestellten Ausführungsform umfasst das Gesamtsystem 1 der erfindungsgemäßen Sehhilfe eine Brille 2 und einen Pocketcomputer 3, der als separate Einheit beispielsweise am Körper des Benutzers getragen werden kann. Die Brille 2 und der Pocketcomputer 3 sind über eine Datenleitung 4 miteinander verbunden, die optional auch eine Energieleitung zur Übertragung von Energie vom Pocketcomputer 3 an die Brille 2 umfassen kann.

Das erfindungsgemäße Sehhilfe-System umfasst elektronischen Mittel zur elektronischen Bearbeitung der erfassten Bilddaten. Diese elektronischen Mittel sind vorzugsweise in einer im Sehhilfe-System integrierten Recheneinheit und/oder in der separaten Einheit 3 untergebracht, die mit der Kamera über die Datenleitung 4 Bilddaten und/oder Steuerungsdaten austauschen kann. Bei der in Figur 1 dargestellten Ausführungsform sind in der separaten Komponente bzw. dem Pocketcomputer 3 elektronische Bauteile enthalten, welche die von der Kamera 7 erfassten und über die Datenleitung 4 übermittelten Bilddaten elektronisch verarbeiten können. Die von den elektronischen Bauteilen in dem Pocketcomputer 3 bearbeiteten Bilddaten können auf einer Anzeige (nicht dargestellt) sichtbar gemacht und dort vom Benutzer eingesehen werden.

Bei einer Anwendung der erfindungsgemäßen Sehhilfe zur Unterstützung eines sehbehinderten Benutzers, können die von den elektronischen Bauteilen in dem Pocketcomputer 3 bearbeiteten Bilddaten über die Datenleitung 4 wieder an die Brille 2 übermittelt und an die in dem Auge 5 des Benutzers implantierte mikroelektronische Stimulations-Vorrichtung 6 weitergeleitet werden. Die Übertragung der Bilddaten von der Brille 2 an die mikroelektronische Stimulations-Vorrichtung 6 erfolgt beispielsweise drahtlos über eine induktive und/oder optoelektronische Schnittstelle. Die mikroelektronische Stimulations-Vorrichtung 6 wandelt die empfangenen Bilddaten in elektrische Stimulations-Impulse um und gibt diese an die Ganglienzellen der Retina und den Sehnerv ab, wodurch dem Benutzer ein entsprechendes Bild visualisiert wird.

Neben der realistischen Wiedergabe des von der Kamera 7 erfassten Bildes, können mit dem erfindungsgemäßen Sehhilfe-System bestimmte Objekte in dem erfassten Bild durch zusätzliche Informationen besonders hervorgehoben und dem Benutzer visualisiert werden. Die Hervorhebung bestimmter Objekte kann beispielsweise durch Verwendung unterschiedlicher Graustufen oder durch Verwendung bestimmter Farben für die Visualisierung der betreffenden Objekte erfolgen. Ferner können insbesondere Objekte in unmittelbarer Umgebung des Benutzers durch die Visualisierung von Informationen zur Entfernung der betreffenden Objekte speziell hervorgehoben werden. So können beispielsweise in der näheren Umgebung befindliche Objekte in einer anderen Farbe oder Graustufe dargestellt werden als weiter entfernt liegende Objekte. Zusätzlich oder alternativ können weiter entfernte Objekte aus dem erfassten Bild ausgeblendet werden. Ferner kann die Umgebung des Benutzers beispielsweise über eine Umrissdarstellung visualisiert werden.

Bei einer Anwendung der erfindungsgemäße Sehhilfe in Verbindung mit einem oben beschriebenen Retina-Implantat-System zur Unterstützung eines sehbehinderten Benutzers kann im Auge 5 des Benutzers eine mikroelektronische Stimulations-Vorrichtung 6 implantiert sein, welche die von der externen Kamera 7 erfassten Bilddaten empfängt. Die mikroelektronische Stimulations-Vorrichtung 6 ist im Bereich der Netzhaut (Retina) implantiert und umfasst eine Vielzahl von Stimulations-Elektroden, die elektrische Stimulations-Impulse auf die Retina entsprechend den empfangenen Bilddaten abgeben. Auf diese Weise wird das durch die Kamera 7 erfasste Bild in elektrische Signale umgesetzt und über Stimulations-Elektroden der Stimulations-Vorrichtung 6 mittels elektrischer Stimulations-Impulse an die Ganglienzellen der Retina und den Sehnerv abgegeben, um so das Sehvermögen des Benutzers wiederherzustellen bzw. zu verbessern.

Die Brille 2 und die separate Einheit bzw. der Pocketcomputer 3 des Sehhilfe-Systems 1 sind über eine drahtgebundene Datenleitung und/oder Energieleitung 4 miteinander verbunden und können über diesen Kommunikationsweg in beiden Richtungen Daten übertragen. In der einen Richtung können die von der elektronischen Kamera 7 in der Brille erfassten Bilddaten an den Pocketcomputer 3 übermittelt werden und in der anderen Richtung kann der Pocketcomputer 3 beispielsweise Steuerungsdaten oder Simulationsdaten an die Kamera 7 übermitteln. Diese von der separaten Komponente 3 an die Kamera 7 gesendeten Steuerungsdaten dienen beispielsweise der Einstellung der Schärfe, der Ausrichtung, des Fokus bzw. des Zooms der Kamera 7, der Auswahl oder der Vergrößerung eines bestimmten Bildausschnitts, den die Kamera 7 erfassen soll.

Figur 2 zeigt eine schematische Darstellung des Sehhilfe-Systems gemäß einer weiteren bevorzugten Ausführungsform der vorliegenden Erfindung mit einem drahtlosen Kommunikationsweg. Der Aufbau der in Figur 2 dargestellten Ausführungsform des erfindungsgemäßen Sehhilfe-Systems entspricht im Wesentlichen dem Aufbau der in Figur 1 dargestellten Ausführungsform, so dass auf die Beschreibung zu Figur 1 Bezug genommen werden kann. Im Unterschied zu der zuvor beschriebenen Ausführungsform, ist bei der Figur 1 dargestellten Ausführungsform anstelle der Drahtleitung 4 eine drahtlose Datenleitung 8 vorgesehen. Eine solche drahtlose Datenleitung 8 kann beispielsweise als bidirektionale Funkverbindung zwischen der Brille 2 und der separaten Einheit bzw. dem Pocketcomputer 3 des Sehhilfe-Systems 1 ausgebildet sein. Über die bidirektionale drahtlose Datenleitung 8 können die Brille 2 und die separate Einheit bzw. der Pocketcomputer 3 des Sehhilfe-Systems 1 miteinander kommunizieren und Bilddaten oder Steuerungsdaten austauschen.

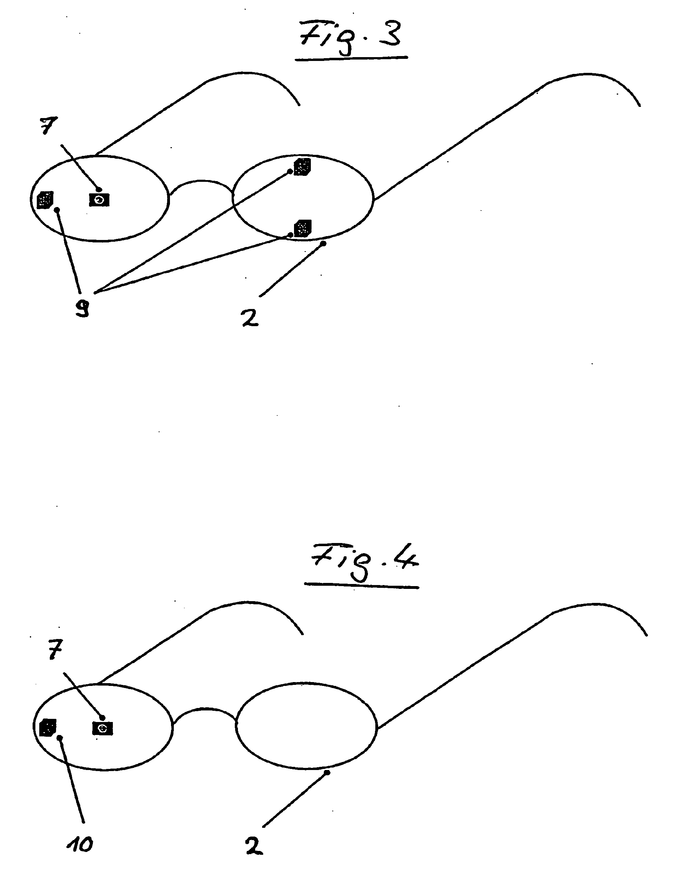

Figur 3 zeigt eine schematische Darstellung des Sehhilfe-Systems gemäß einer weiteren bevorzugten Ausführungsform der vorliegenden Erfindung mit dreidimensionalen Beschleunigungs-Sensoren 9. Dabei sind an der Brille 2 sowohl die Kamera 7 als auch drei Beschleunigungs-Sensoren 9 angeordnet und auf diese Weise in den Bewegungen miteinander gekoppelt. Die Beschleunigungs-Sensoren 9 messen die translatorische Beschleunigung [*ẍ ÿ z̈*] der Kamera 7 in den drei Raumrichtungen [*x, y, z*]. Zusätzlich kann aus den translatorischen Beschleunigungswerten der Sensoren 9 die Winkelbeschleunigung [*ẍ ÿ z̈* ϕ̈*ₓ* ϕ̈*_{y}* ϕ̈*_{z}*] der Kamera 7 um die drei Raumachsen ermittelt werden.

Figur 4 zeigt eine schematische Darstellung des Sehhilfe-Systems gemäß einer weiteren bevorzugten Ausführungsform der vorliegenden Erfindung mit einem sechsdimensionalen Beschleunigungs-Sensor. Der sechsdimensionale Beschleunigungs-Sensor 10 ist wie die Kamera 7 an der Brille 2 angeordnet und vollzieht somit die gleichen Bewegungen und Beschleunigungen wie die Kamera 7. Dieser sechsdimensionale Beschleunigungs-Sensor 10 ist in der Lage, sowohl die translatorische Beschleunigung [*ẍ ÿ z̈*] der Kamera 7 als auch die Winkelbeschleunigung [*ẍ ÿ z̈* ϕ̈*ₓ* ϕ̈*_{y}* ϕ̈*_{z}*] der Kamera um die drei Raumachsen zu messen.

Anhand der von den Beschleunigungs-Sensoren 9 und den Winkelbeschleunigungs-Sensoren 10 gelieferten Messwerte kann eine Veränderung der räumlichen Ausrichtung der Kamera 7 relativ zu einer vorhergehenden räumlichen Ausrichtung der Kamera 7 bestimmt werden. Dazu wird die räumliche Position oder die Ausrichtung der Kamera 7 relativ zu einer vorhergehenden Position oder Ausrichtung der Kamera 7 mittels der elektronischen Mittel des Sehhilfe-Systems 1, durch ein Trägheitsnavigationsverfahren bestimmt.

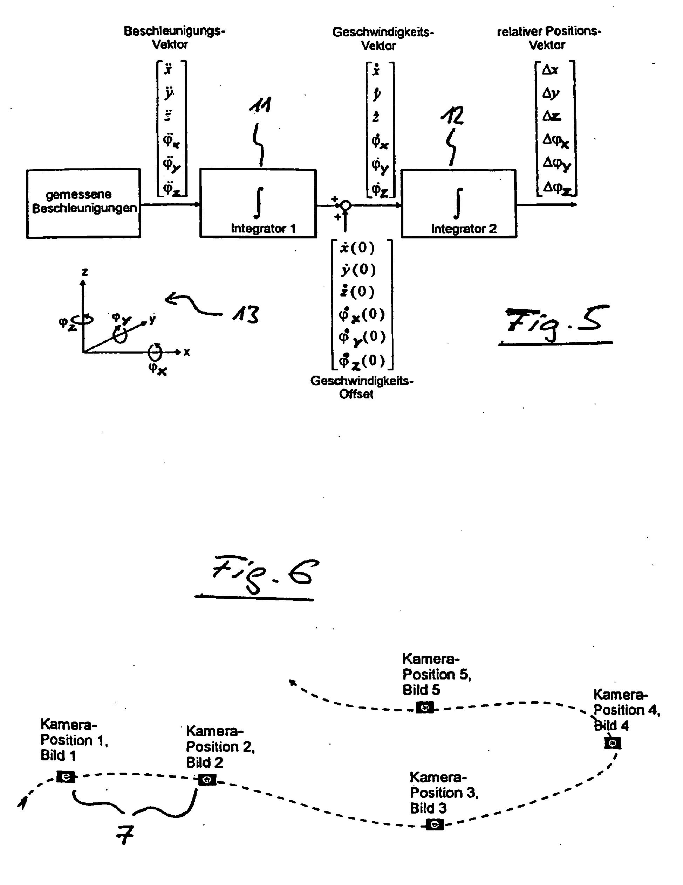

Figur 5 zeigt eine schematische Darstellung der in dem erfindungsgemäßen Sehhilfe-System durchgeführten Berechnung zur Positionsbestimmung des Sehhilfe-Systems gemäß einer bevorzugten Ausführungsform der vorliegenden Erfindung. Die elektronischen Mittel des erfindungsgemäßen Sehhilfe-Systems 1 umfassen elektronische Integratoren 11 und 12, die in der Lage sind, eine mathematische Integration numerisch auszuführen. Wie in Figur 5 dargestellt, lässt sich aus den von den Beschleunigungs-Sensoren gemessenen orthogonalen Beschleunigungsmesswerten ein sechsdimensionaler Beschleunigungsvektor erstellen, der die drei translatorischen Beschleunigungswerte und die drei Winkelbeschleunigungswerte enthält. Die Indices x, y, z der Vektoreinträge beziehen sich jeweils auf die orthogonalen Raumachsen des Inertialsystems 13 von der Kamera 7. Die Winkelausrichtung der Kamera kann auch durch Auswertung vorhergehender Winkelpositionswerte, durch einen Kompass, eine Hallsonde und/oder einen gravitationsbasierten Neigungssensor bestimmt werden.

Die elektronischen Bauteile des Pocketcomputers 3 sind so ausgebildet, dass sie die numerische Integration der von den Beschleunigungs-Sensoren gelieferten Messwerte durchführen können, um die räumlichen Position und Ausrichtung der Kamera zu bestimmen. Dazu wird aus den sechs orthogonalen Beschleunigungsmesswerten des Beschleunigungsvektors mittels des ersten Integrators 11 ein sechsdimensionaler Geschwindigkeitsvektor generiert, der drei translatorische Geschwindigkeitswerte und drei Rotations-Geschwindigkeitswerte enthält. Über die Veränderung der erfassten Bilddaten und unter Berücksichtigung der von den mit der Kamera gekoppelten Beschleunigungs-Sensoren ermittelten Beschleunigungsmesswerten kann folglich auch die aktuelle Geschwindigkeit der Kamera bestimmt werden. Hier kann durch einfache Vektoraddition eines sechsdimensionalen Geschwindigkeits-Offsetvektors ein Geschwindigkeits-Offset berücksichtigt und so aus den Berechnungen annulliert werden. Es können auch Eingabemittel vorgesehen sein, durch die der Messwert der Beschleunigungs-Sensoren und/oder Winkelbeschleunigungs-Sensoren auf Null gesetzt wird.

Mittels des zweiten Integrators 12 wird aus dem Geschwindigkeitsvektor ein sechsdimensionaler relativer Positionsvektor generiert, der die Differenz einer vorhergehenden Position der Kamera zu einer nachfolgenden Kameraposition angibt. Dieser sechsdimensionale relative Positionsvektor enthält drei Ortsdifferenzkoordinaten (Δx, Δy, Δz), welche die Differenz der Ortskoordinaten (x, y, z) der vorhergehenden Kameraposition zur nachfolgenden Kameraposition angeben, und drei Winkeldifferenzkoordinaten (Δϕₓ, Δϕ_{y}, Δϕ_{z}), welche die Differenz der Winkelposition (ϕₓ, ϕ_{y}, ϕ_{z}) der vorhergehenden Kameraposition zur nachfolgenden Kameraposition angeben. Die Integration kann in diskreten Zeitschritten erfolgen oder auch kontinuierlich durchgeführt werden, wobei die zeitliche Intergrationsschrittweite bei der Integration der Beschleunigungsmesswerte kleiner sein sollte als die Zeitspanne, innerhalb der sich die Beschleunigung der Kamera wesentlich ändert.

Figur 6 zeigt eine schematische Darstellung einer Bewegungsspur mit einer Anzahl von unterschiedlichen aufeinander folgenden Positionen des Sehhilfe-Systems nach der vorliegenden Erfindung. Mit dem erfindungsgemäßen Sehhilfe-System kann der Kamera 7 eine Sequenz von mindestens zwei Bildern bei jeweils unterschiedlicher Position der Kamera 7 erfasst werden oder eine Sequenz von Bildern in festen Zeitintervallen bei jeweils unterschiedlicher Position der Kamera aufgenommen werden. Aus einer solchen Sequenz in relativ kurzer Zeitfolge hintereinander erfasster Bilder mit unterschiedlichen Kamerapositionen kann mittels der elektronischen Mittel, eine stereoskopische Wiedergabe der erfassten Bilder, d.h. eine dreidimensionale Wiedergabe der erfassten Umgebung berechnet und visualisiert werden.

Dazu wird aus der Sequenz der erfassten Bilder aus unterschiedlichen Kamerapositionen mittels der elektronischen Mittel die räumliche Position und/oder Ausrichtung der Kamera 7 berechnet. Jedem erfassten Bild der Sequenz kann unter Verwendung von Ortsdifferenzkoordinaten und Winkeldifferenzkoordinaten die relative Position der Kamera 7 in Bezug auf die Kameraposition bzw. die Position des Benutzers des Sehhilfe-System bei der Erfassung des vorangehenden oder nachfolgenden Bildes zugeordnet werden. Dabei können ältere Bilder einer Sequenz von erfassten Bildern in ihrem Einfluss auf die Berechnungen zunehmend schwächer gewichtet werden oder keinen Einfluss mehr auf die Berechnungen haben. Auf diese kann mit dem erfindungsgemäßen Sehhilfe-System mit nur einer Kamera 7 anhand einer Sequenz von seriell erfassten Bildern aus unterschiedlichen Kamerapositionen eine dreidimensionale Wiedergabe der erfassten Bilder und der darin befindlichen Objekte berechnet und visualisiert werden.

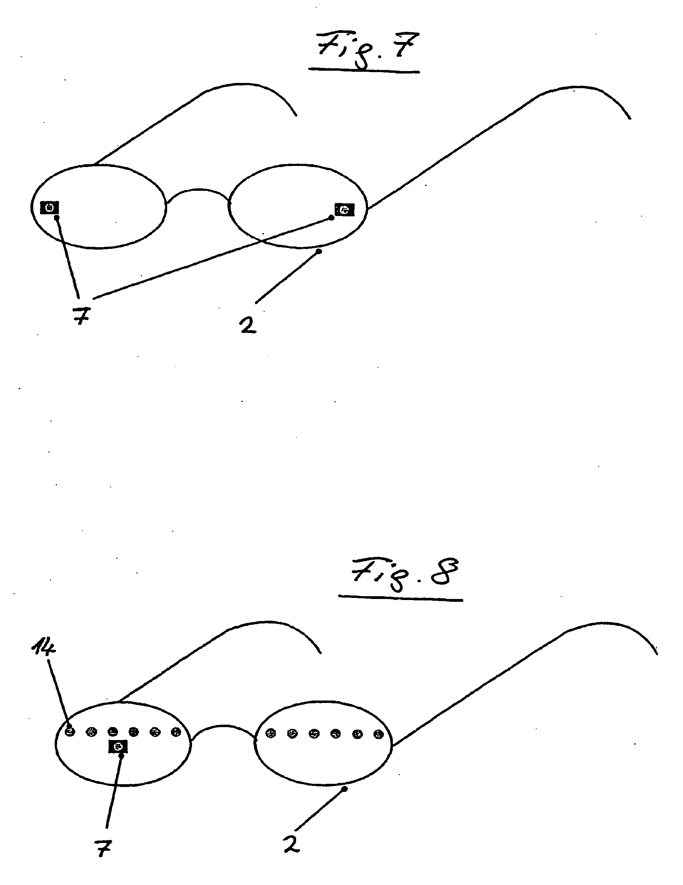

Figur 7 zeigt eine schematische Darstellung des Sehhilfe-Systems gemäß einer weiteren bevorzugten Ausführungsform der vorliegenden Erfindung mit zwei Kameras 7. Die zweite Kamera ist an einer von der ersten Kamera entfernten Position in der Brille 2 integriert. Mit Hilfe der beiden Kameras 7 können zwei Bilder gleichzeitig erfasst und mittels damit ein stereoskopisches Bild erstellt werden. Anhand der erfassten Stereobilder kann mittels der elektronischen Mittel des Sehhilfe-Systems eine dreidimensionale Wiedergabe des erfassten Bildes und der darin befindlichen Objekte berechnet und dem Benutzer visualisiert werden. Die Berechnung der dreidimensionalen Wiedergabe des erfassten Bildes kann unter Anwendung des Bündelausgleichsverfahrens durchführt werden.

Figur 8 zeigt eine schematische Darstellung des Sehhilfe-Systems gemäß einer weiteren bevorzugten Ausführungsform der vorliegenden Erfindung mit Sensor-Arrays. Bei dieser Ausführungsform sind mehrere Abstandssensoren an der Brille 2 vorgesehen, die in Form eines eindimensionalen oder mehrdimensionalen Arrays 14 angeordnet sind. Die Sensoren können dazu dienen, die Entfernung von Objekten und/oder Hindernissen in dem erfassten Bild zu bestimmen und als zusätzliche Information dem Sehhilfe-System zur Verfügung zu stellen. Die Abstandssensoren können als Ultraschallsensor oder rotierender Laserscanner im sichtbaren oder unsichtbaren Wellenlängenbereich ausgebildet sein. Es können auch andere Typen von Sensoren oder Aktoren in der Brille 2 vorgesehen sein, wie z.B. Ultraschallsensoren, Infrarot-Sensoren, Photosensoren, Laser oder Kombinationen daraus.

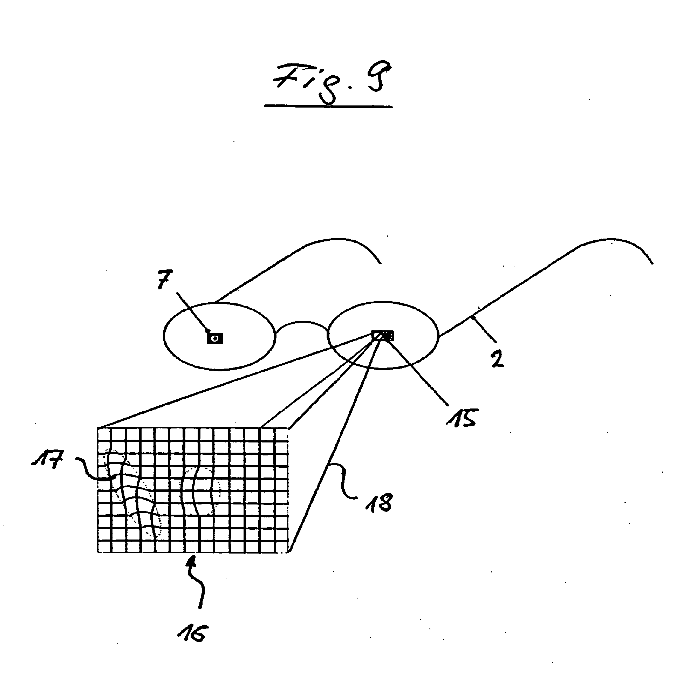

Figur 9 zeigt eine schematische Darstellung einer weiteren bevorzugten Ausführungsform des erfindungsgemäßen Sehhilfe-Systems. Bei dieser bevorzugten Ausführungsform ist das Sehhilfe-System mit einer Lichtquelle 15 ausgestattet, um den von der Kamera zu erfassenden Bildbereich mit Licht 18 im sichtbaren oder unsichtbaren Wellenlängenbereich, mit Infrarot-Licht oder mit infrarotem oder sichtbarem Laserlicht 18 zu beleuchten, während die Kamera 7 des Sehhilfe-Systems in dem entsprechenden Wellenlängenbereich sensitiv ist. Mittels einer solchen Lichtquelle 15 kann der von der Kamera zu erfassende Bildbereich mit einem homogenen oder strukturierten Streifenmuster beleuchtet werden. Zur Projektion eines strukturierten Streifenmusters 16 auf den von der Kamera 7 zu erfassenden Bildbereich kann auch ein Laserprojektor mit einer geeigneten Ablenkeinheit verwendet werden, ein Gitterprojektor oder eine Projektionsmaske, die im Strahlengang einer Lichtquelle positioniert ist. Es können auch mit Laserlicht Interferenz-Effekte erzeugt und auf den von der Kamera zu erfassenden Bildbereich ein Interferenz-Muster projiziert werden.

Figur 9 zeigt eine schematische Darstellung des Sehhilfe-Systems, das mit einem Gitter-Projektor ausgestattet ist. Der Gitter-Projektor belichtet den von der Kamera zu erfassenden Bildbereich mit einem Streifen- oder Gittermuster, das auf die Objekte in dem Bildbereich fällt und davon reflektiert wird. Dadurch ergeben sich Verzerrungen, des Streifen- oder Gittermusters aus denen Rückschlüsse auf die Position, Größe und Lage der Objekte in dem erfassten Bildbereich gezogen werden können. Mit Hilfe der elektronischen Mittel des Sehhilfe-Systems 1 kann aus den Verzerrungen, Verbiegungen, Verschiebungen oder sonstigen Veränderungen des Streifen- oder Gittermusters eine dreidimensionale Wiedergabe des erfassten Bildes und der darin befindlichen Objekte erstellt und visualisiert werden.

Das Sehhilfe-System kann abwechselnd ein Bild des zu erfassenden Bildbereichs unter Beleuchtung mit einem Streifenmuster 16 und ein Bild des zu erfassenden Bildbereichs ohne Streifenmuster 16 aufnehmen und die Bilddaten der erfassten Bilder mittels der elektronischen Mittel miteinander kombinieren oder vergleichen. Der zu erfassende Bildbereich kann mittels eines spalten- und zeilenweise gesteuerten Laserstrahls 18 abgetastet werden, um zusätzliche Bilddaten und/oder Informationen über die Entfernung von Objekten in dem erfassten Bild zu erhalten. Dabei kann das erfindungsgemäße Sehhilfe-System 1 mit einem Photodetektor ausgestattet sein, der die räumliche Position von Objekten im erfassten Bild und/oder deren Abstand zur Kamera bestimmt, indem der Photodetektor eine Laufzeitmessung des vom betreffenden Bildpunkt reflektierten Laserlichtes 18 durchführt.

### Liste der Bezugszeichen

- 1: Sehhilfe-System
- 2: Brille
- 3: separate elektronische Einheit bzw. Pocketcomputer
- 4: drahtgebundene Datenleitung und/oder Energieleitung zwischen 2 und 3
- 5: Auge des Benutzers
- 6: implantierbare Stimulations-Vorrichtung
- 7: Kamera
- 8: drahtlose Datenleitung zwischen 2 und 3
- 9: Sensoren zur Messung der translatorischen oder Winkelbeschleunigung
- 10: Sensor zur Messung der translatorischen und Winkelbeschleunigung
- 11: elektronische Mittel bzw. elektronischer Integrator
- 12: elektronische Mittel bzw. elektronischer Integrator II
- 13: Inertialsystem der Kamera
- 14: Array von Sensoren
- 15: Lichtquelle bzw. Gitterprojektor
- 16: projiziertes Gitter- oder Steifenmuster
- 17: Verzerrung im reflektierten Gitter- oder Steifenmuster
- 18: Lichtstrahlen bzw. Laserlichtstrahlen

## Patentansprüche

1. Sehhilfe-System umfassend mindestens eine erste Kamera (7) zum Erfassen eines Bildes mit einer Vielzahl von Bilddaten und elektronische Mittel (11, 12) zum Bearbeiten der erfassten Bilddaten, wobei die elektronischen Mittel (11, 12) ausgelegt sind, bei der Wiedergabe des bearbeiteten Bildes zusätzliche Informationen bezüglich der räumlichen Position und/oder bestimmter Attribute von Objekten in dem erfassten Bild zu visualisieren, die mit Objekten in dem erfassten Bild assoziiert sind, wobei Informationen bezüglich des Abstands zwischen der Kamera (7) und Objekten in dem erfassten Bild bei der Wiedergabe des bearbeiteten Bildes mit den Objekten assoziiert sind, **dadurch gekennzeichnet,**
**dass** das Sehhilfe-System ferner eine im Körper am Sehorgan (5) implantierbare Stimulations-Vorrichtung (6) zur Wiedergabe des bearbeiteten Bildes durch entsprechende Stimulierung des Sehorgans (5) umfasst,
wobei die elektronischen Mittel (11, 12) ausgelegt sind, eine tiefenselektive Darstellung des erfassten Bildes zu ermöglichen.

2. Sehhilfe-System nach einem der vorangehenden Ansprüche, wobei die zusätzlichen Informationen bezüglich der Position von Objekten in dem erfassten Bild bei der Wiedergabe des bearbeiteten Bildes durch visuelle Hervorhebung bestimmter Objekte angegeben werden, bevorzugt durch Verwendung unterschiedlicher Graustufen und/oder bestimmter Farben für die Visualisierung bestimmter Objekte.

3. Sehhilfe-System nach einem der vorangehenden Ansprüche, wobei Objekte in dem erfassten Bild bei der Wiedergabe des bearbeiteten Bildes durch die Visualisierung von Informationen zur Entfernung der betreffenden Objekte in Bezug auf die Position vom Benutzer des Sehhilfe-Systems (1) durch besondere Visualisierung gekennzeichnet werden.

4. Sehhilfe-System nach einem der vorangehenden Ansprüche, wobei in Bezug auf die Position vom Benutzer des Sehhilfe-Systems (1) Objekte im erfassten Bild mit geringerer Entfernung durch die Visualisierung mit unterschiedlichen Graustufen und/oder unterschiedlichen Farben gegenüber Objekten in größerer Entfernung bei der Wiedergabe des bearbeiteten Bildes gekennzeichnet werden.

5. Sehhilfe-System nach einem der vorangehenden Ansprüche, wobei in Bezug auf die Position vom Benutzer des Sehhilfe-Systems (1) weiter entfernte Objekte im erfassten Bild bei der Wiedergabe des bearbeiteten Bildes ausgeblendet werden.

6. Sehhilfe-System nach einem der vorangehenden Ansprüche, wobei jedem Bildpunkt des bearbeiteten Bildes ein Bildpunktvektor [Pᵢ = (xᵢ, yᵢ, zᵢ, Eᵢ)] zugeordnet wird, der dreidimensionale Raumkoordinaten [xᵢ, yᵢ, zᵢ] sowie eine Eigenschaftsvariable Eᵢ umfasst, die bestimmte Attribute des betreffenden Bildpunktes repräsentiert.

7. Sehhilfe-System nach einem der vorangehenden Ansprüche, wobei eine dreidimensionale Wiedergabe des erfassten Bildes Gitterpunkte [Pᵢ = (xᵢ, yᵢ, zᵢ)] enthalten, die mit bestimmten Bildpunkten des erfassten Bildes korrespondieren und durch Linien zwischen benachbarten Gitterpunkten ein Gitternetz aufgespannt wird.

8. Sehhilfe-System nach einem der vorangehenden Ansprüche, wobei bei einem bestimmten Betriebsmodus nur solche Objekte im erfassten Bild bei der Wiedergabe des bearbeiteten Bildes visualisiert werden, die sich im in einem bestimmten Erfassungsbereich des Sehhilfe-Systems (1) befinden.

9. Sehhilfe-System nach einem der vorangehenden Ansprüche, wobei am Sehhilfe-System (1) unterschiedliche Betriebsmodi einstellbar sind, nach denen das Sehhilfe-System (1) das erfasste Bild bearbeitet und das bearbeitete Bild wiedergibt, und wobei die Auswahl der Betriebsmodi über Bedienelemente am Sehhilfe-System (1) oder über eine Fernbedienung erfolgt.

10. Sehhilfe-System nach dem vorangehenden Anspruch, wobei bei einem bestimmten Betriebsmodus der optische Erfassungsbereich des Sehhilfe-Systems (1) wiederholt erweitert und danach wieder verringert wird, so dass kontinuierlich räumliche Schnittbilder mit unterschiedlichem Erfassungsbereich visualisiert werden.

11. Sehhilfe-System nach einem der vorangehenden Ansprüche, wobei mittels der elektronischen Mittel (11, 12) anhand der erfassten Bilddaten eine Darstellung aus der Vogelperspektive oder anderen Perspektiven oder Zoomstufen des erfassten Bildes und der darin befindlichen Objekte erstellt und visualisiert wird.

12. Sehhilfe-System nach einem der vorangehenden Ansprüche, wobei mittels der elektronischen Mittel (11, 12) anhand der erfassten Bilddaten ein Grundriss des erfassten Bildes und der darin befindlichen Objekte erstellt und visualisiert wird.

13. Sehhilfe-System nach einem der vorangehenden Ansprüche, wobei mittels der elektronischen Mittel (11, 12) anhand der erfassten Bilddaten eine dreidimensionale Wiedergabe des erfassten Bildes und der darin befindlichen Objekte erstellt und visualisiert wird.

14. Sehhilfe-System nach einem der vorangehenden Ansprüche, das ferner einen Datenspeicher umfasst, in dem eine Bild-Bibliothek mit einer Anzahl von Bild-Mustern gespeichert werden können, und wobei die elektronischen Mittel (11, 12) dazu ausgebildet sind, eine Bild-Mustererkennung durchzuführen, wobei die Objekte in dem erfassten Bild mit vorgegebenen Bild-Mustern verglichen und entsprechend zugeordnet werden.

15. Sehhilfe-System nach dem vorangehenden Anspruch, bei dem die Erstellung einer dreidimensionalen Wiedergabe zumindest zwei Bilder von räumlich unterschiedlichen Positionen der Kamera (7) unter Berücksichtigung der räumlich unterschiedlichen Positionen und der relativen Winkel-Orientierung der Kamera (7) erfasst und von den elektronischen Mitteln (11, 12) bearbeitet werden.

16. Sehhilfe-System nach einem der vorangehenden Ansprüche, wobei die Kamera (7) mit mindestens einem Beschleunigungs-Sensor (9) gekoppelt ist, der die translatorische Beschleunigung [*ẍ ÿ z̈*] der Kamera (7) in den drei Raumrichtungen [*x, y, z*] ermittelt und in Abhängigkeit von der Anzahl und Anordnung der Beschleunigungs-Sensoren (9) auch die Winkelbeschleunigungen [*ẍ ÿ* z ϕₓ*,* ϕ̈*_{y}* ϕ̈*_{z}*] der Kamera (7) um die drei Raumachsen [*x, y, z*] ermitteln kann.

17. Sehhilfe-System nach einem der vorangehenden Ansprüche, wobei die Kamera (7) mit mindestens einem Winkelbeschleunigungs-Sensor (10) gekoppelt ist, der die Winkelbeschleunigung [*ẍ ÿ z̈* ϕ̈*ₓ* ϕ̈*_{y}* ϕ̈*_{z}*] der Kamera (7) um die drei Raumachsen [*x, y, z*] ermittelt.

18. Sehhilfe-System nach einem der vorangehenden Ansprüche, wobei mittels der elektronischen Mittel (11, 12) anhand der von einer Anzahl von Winkelbeschleunigungs-Sensoren (10) gelieferten Winkelbeschleunigungsmesswerte eine Veränderung der räumlichen Ausrichtung der Kamera (7) relativ zu einer vorhergehenden räumlichen Ausrichtung der Kamera (7) bestimmt wird; und/oder
wobei das Sehhilfe-System (1) elektronische Integratoren (11, 12) zur Integration der von einer Anzahl von Beschleunigungs-Sensoren (9, 10) gelieferten Beschleunigungsmesswerte und zur Berechnung der räumlichen Position und Ausrichtung der Kamera (7) umfasst.

19. Sehhilfe-System nach einem der vorangehenden Ansprüche, wobei eine Sequenz von mindestens zwei Bildern bei jeweils unterschiedlicher Position der Kamera (7) erfasst wird, bevorzugt in festen Zeitintervallen.

20. Sehhilfe-System nach einem der vorangehenden Ansprüche, wobei aus der Sequenz der erfassten Bilder aus unterschiedlichen Kamerapositionen mittels der elektronischen Mittel (11, 12) eine dreidimensionale oder stereoskopische Wiedergabe der erfassten Bilder und der darin befindlichen Objekte berechnet und visualisiert wird; und/oder
wobei aus der Sequenz der erfassten Bilder aus unterschiedlichen Kamerapositionen mittels der elektronischen Mittel (11, 12) die räumliche Position und/oder Ausrichtung der Kamera (7) berechnet werden.

21. Sehhilfe-System nach einem der vorangehenden Ansprüche, wobei die zumindest eine Kamera (7) in einer Brille (2) integriert ist, die vom Benutzer als normale Brille getragen werden kann.

22. Sehhilfe-System nach einem der vorangehenden Ansprüche, wobei eine zweite Kamera (7) vorgesehen ist, die zusammen mit der ersten Kamera (7) Stereobilder erfasst, wobei die zweite Kamera (7) bevorzugt in der Brille (2) an einer von der ersten Kamera (7) entfernten Position in der Brille (2) integriert ist.

23. Sehhilfe-System nach einem der vorangehenden Ansprüche, wobei eine aktuelle Geschwindigkeit der Kamera (7) über die Veränderung der erfassten Bilddaten unter Berücksichtigung der von den mit der Kamera (7) gekoppelten Beschleunigungs-Sensoren (9, 10) ermittelten Beschleunigungsmesswerte bestimmt wird.

24. Sehhilfe-System nach einem der vorangehenden Ansprüche, wobei die Winkelausrichtung der Kamera (7) durch Auswertung vorhergehender Winkelpositionswerte, durch einen Kompass, eine Hallsonde und/oder einen gravitationsbasierten Neigungssensor bestimmt wird.

25. Sehhilfe-System nach einem der vorangehenden Ansprüche, wobei mindestens ein Abstandssensor vorgesehen ist, um die Entfernung von Objekten und/oder Hindernissen in dem erfassten Bild zu bestimmen, und wobei der Abstandssensor vorzugsweise als Ultraschallsensor oder rotierender Laserscanner im sichtbaren oder unsichtbaren Wellenlängenbereich ausgebildet ist.

26. Sehhilfe-System nach dem vorangehenden Anspruch, wobei das Sehhilfe-System (1) eine Lichtquelle umfasst, um den von der Kamera (7) zu erfassenden Bildbereich mit Licht (18) im sichtbaren oder unsichtbaren Wellenlängenbereich, mit Infrarot-Licht oder mit Laserlicht (18) zu beleuchten, und die Kamera (7) des Sehhilfe-Systems (1) in dem entsprechenden Wellenlängenbereich sensitiv ist.

27. Sehhilfe-System nach dem vorangehenden Anspruch, wobei das Sehhilfe-System auf den von der Kamera (7) zu erfassenden Bildbereich Licht (18) mit einem homogenen oder strukturierten Streifenmuster (16) projiziert, und wobei mittels der elektronischen Mittel (11, 12) aus Verzerrungen, Verbiegungen, Verschiebungen oder sonstige Veränderungen (17) des Streifenmusters (16) im erfassten Bild eine dreidimensionale Wiedergabe des erfassten Bildes und der darin befindlichen Objekte erstellt und visualisiert wird.

28. Sehhilfe-System nach einem der vorangehenden Ansprüche, wobei das Sehhilfe-System zur Beleuchtung des von der Kamera (7) zu erfassenden Bildbereichs mit einem Streifenmuster (16) einen Laserprojektor mit Ablenkeinheit, einen Gitterprojektor (15) oder eine Projektionsmaske umfasst, die im Strahlengang einer Lichtquelle positioniert ist.

29. Sehhilfe-System nach einem der vorangehenden Ansprüche, wobei das Sehhilfe-System mit Laserlicht (18) Interferenz-Effekte erzeugt und auf den von der Kamera (7) zu erfassenden Bildbereich ein Interferenz-Muster (16) projiziert.

30. Sehhilfe-System nach einem der vorangehenden Ansprüche, wobei das Sehhilfe-System abwechselnd ein Bild des zu erfassenden Bildbereichs unter Beleuchtung mit einem Streifenmuster (16) und ein Bild des zu erfassenden Bildbereichs ohne Beleuchtung erfasst und die Bilddaten der erfassten Bilder mittels der elektronischen Mittel (11, 12) miteinander kombiniert oder vergleicht.

31. Sehhilfe-System nach einem der vorangehenden Ansprüche, wobei der zu erfassende Bildbereich mittels eines Laserstrahls abgetastet wird, um Bilddaten und/oder Informationen über die Entfernung von Objekten in dem erfassten Bild zu erhalten.

## Claims

1. Vision aid system comprising at least one first camera (7) for acquisition of an image having a plurality of image data, and comprising electronic means (11, 12) for processing the acquired image data, wherein the electronic means (11, 12) are adapted to visualize additional information regarding the spatial position and/or particular attributes of objects in the acquired image in the reproduction of the processed image, which information is associated with objects in the acquired image, wherein information regarding the distance between the camera (7) and objects in the acquired image is associated with the objects in the reproduction of the processed image,
**characterized in that**
the vision aid system further comprises a stimulation device (6) implantable in the body at the vision organ (5) for reproduction of the processed image through corresponding stimulation of the vision organ (5),
wherein the electronic means (11, 12) are adapted to allow for a depth-selective representation of the acquired image.

2. Vision aid system according to any one of the preceding claims, wherein the additional information regarding the position of objects in the acquired image is specified in the reproduction of the processed image through visual accentuation of particular objects, preferably through use of different grey levels and/or particular colors for the visualization of particular objects.

3. Vision aid system according to any one of the preceding claims, wherein objects in the acquired image are **characterized in** the reproduction of the processed image by the visualization of information on the distance of the related objects in relation to the position of the user of the vision aid system (1) by particular visualisation.

4. Vision aid system according to any one of the preceding claims, wherein, in relation to the position of the user of the vision aid system (1), objects in the acquired image with lesser distance are **characterized in** the reproduction of the processed image by the visualization with different grey levels and/or different colors compared to objects at greater distance.

5. Vision aid system according to any one of the preceding claims, wherein more remote objects in the acquired image in relation to the position of the user of the vision aid system (1) are masked out in the reproduction of the processed image.

6. Vision aid system according to any one of the preceding claims, wherein each pixel of the processed image is assigned a pixel vector [Pᵢ = (xᵢ, yᵢ, zᵢ, Eᵢ)], which comprises three-dimensional spatial coordinates [xᵢ, yᵢ, zᵢ] as well as a characteristic variable Eᵢ that represents particular attributes of the related pixel.

7. Vision aid system according to any one of the preceding claims, wherein a three-dimensional reproduction of the acquired image contains grid points [Pᵢ = (xᵢ, yᵢ, zᵢ)], which correspond to particular pixels of the acquired image, and a grid network is spanned by lines between neighboring grid points.

8. Vision aid system according to any one of the preceding claims, wherein, in a particular operating mode, only those objects in the acquired image that are located in a particular acquisition area of the vision aid system (1) are visualized in the reproduction of the processed image.

9. Vision aid system according to any one of the preceding claims, wherein different operating modes are settable for the vision aid system (1), according to which the vision aid system (1) processes the acquired image and reproduces the processed image, and wherein selection of the operating modes occurs via activation elements on the vision aid system (1) or via a remote control.

10. Vision aid system according to any one of the preceding claims, wherein, in a particular operating mode, the optical acquisition area of the vision aid system (1) is repeatedly enlarged and then reduced again, so that spatial section images with a different acquisition area are continuously visualized.

11. Vision aid system according to any one of the preceding claims, wherein, by means of the electronic means (11, 12) with the acquired image data, a representation or zoom levels of the acquired image and the objects located therein from the bird's-eye perspective or another perspective is generated and visualized.

12. Vision aid system according to any one of the preceding claims, wherein, by means of the electronic means (11, 12) with the acquired image data, a layout view of the acquired image and the objects located therein is generated and visualized.

13. Vision aid system according to any one of the preceding claims, wherein, by means of the electronic means (11, 12) with the acquired image data, a three-dimensional reproduction of the acquired image and the objects located therein is generated and visualized.

14. Vision aid system according to any one of the preceding claims, further comprising a data storage, in which an image library having a number of image patterns can be stored, and wherein the electronic means (11, 12) are configured to carry out an image pattern recognition, wherein the objects in the acquired image are compared with pre-provided image patterns and are associated correspondingly.

15. Vision aid system according to the preceding claim, in which the generation of a three-dimensional reproduction acquires at least two images from spatially different positions of the camera (7) taking account of the spatially different positions and the relative angular orientation of the camera (7) and are processed by the electronic means (11, 12).

16. Vision aid system according to any one of the preceding claims, wherein the camera (7) is coupled with at least one acceleration sensor (9) which ascertains the translational acceleration [*ẍ ÿ z̈*] of the camera (7) in the three spatial directions [x, y, z] and, in dependence upon the number and arrangement of the acceleration sensors (9), can also ascertain the angular accelerations [*ẍ ÿ z̈* ϕ̈*ₓ* ϕ̈*_{y}* ϕ̈*_{z}*] of the camera (7) about the three spatial axes [x, y, z].

17. Vision aid system according to any one of the preceding claims, wherein the camera (7) is coupled with at least one angular acceleration sensor (10) which ascertains the angular acceleration [*ẍ ÿ z̈* ϕ̈*ₓ* ϕ̈*_{y}* ϕ̈*_{z}*] of the camera (7) about the three spatial axes [x, y, z].

18. Vision aid system according to any one of the preceding claims, wherein, by means of the electronic means (11, 12) with the angular acceleration measurement values delivered by a number of angular acceleration sensors (10), a change of the spatial orientation of the camera (7) relative to a previous spatial orientation of the camera (7) is determined; and/or
wherein the vision aid system (1) comprises electronic integrators (11, 12) for integration of the acceleration measurement values delivered by a number of acceleration sensors (9, 10) and for calculation of the spatial position and orientation of the camera (7).

19. Vision aid system according to any one of the preceding claims, wherein a sequence of at least two images at respectively different positions of the camera (7) is acquired, preferably in fixed time intervals.

20. Vision aid system according to any one of the preceding claims, wherein, from the sequence of the acquired images from different camera positions, a three-dimensional or stereoscopic reproduction of the acquired images and of the objects located therein is calculated and visualized by means of the electronic means (11, 12); and/or
wherein, from the sequence of acquired images from different camera positions, the spatial position and/or orientation of the camera (7) are calculated by means of the electronic means (11, 12).

21. Vision aid system according to any one of the preceding claims, wherein the at least one camera (7) is integrated in spectacles (2), which can be worn by a user as normal spectacles.

22. Vision aid system according to any one of the preceding claims, wherein a second camera (7) is provided, which together with the first camera (7) acquires stereo images, wherein the second camera (7) is preferably integrated in the spectacles (2) at a position in the spectacles (2) spaced from the first camera (7).

23. Vision aid system according to any one of the preceding claims, wherein a current velocity of the camera (7) is determined via the change of the acquired images data taking account of the acceleration measurement values ascertained by the acceleration sensors (9, 10) coupled with the camera (7).

24. Vision aid system according to any one of the preceding claims, wherein the angular orientation of the camera (7) is determined by evaluation of previous angular position values, by the compass, by the Hall-probe and/or the gravitation-based inclination sensor.

25. Vision aid system according to any one of the preceding claims, wherein at least one distance sensor is provided to determine the distance of objects and/or obstacles in the acquired images, and wherein the distance sensor is preferably designed as an ultrasound sensor or rotating laser scanner in the visible or invisible wavelength range.

26. Vision aid system according to the previous claim, wherein the vision aid system (1) comprises a light source to illuminate the image area to be acquired by the camera (7) with light (18) in the visible or invisible wavelength range, with infrared light, or with laser light (18), and wherein the camera (7) of the vision aid system (1) is sensitive in the corresponding wavelength range.

27. Vision aid system according to the previous claim, wherein the vision aid system projects light (18) having a homogeneous or structured stripe pattern (16) onto the image area to be acquired by the camera (7), and wherein a three-dimensional reproduction of the acquired image and the objects located therein is generated and visualized by means of the electronic means (11, 12) from distortions, curvatures, displacements or other changes (17) of the stripe pattern (16) in the acquired image.

28. Vision aid system according to any one of the preceding claims, wherein, for illumination of the image area to be acquired by the camera (7) with a stripe pattern (16), the vision aid system comprises a laser projector having deflection unit, a grid projector (15), or a projection mask, which is positioned in the beam path of a light source.

29. Vision aid system according to any one of the preceding claims, wherein the vision aid system generates interference effects with laser light (18) and projects an interference pattern (16) onto the image area to be acquired by the camera (7).

30. Vision aid system according to any one of the preceding claims, wherein the vision aid system alternately acquires an image of the image area to be acquired under illumination with a stripe pattern (16) and an image of the image area to be acquired without illumination, and combines or compares the image data of the acquired images with one another by means of the electronic means (11, 12).

31. Vision aid system according to any one of the preceding claims, wherein the image area to be acquired is scanned by means of a laser beam, to obtain image data and/or information about the distance of objects in the acquired image.

## Revendications

1. Système d'assistance à la vision comprenant au moins une première caméra (7) pour la saisie d'une image avec une multiplicité de données d'image et des moyens électroniques (11, 12) pour le traitement des données d'image saisies, où les moyens électroniques (11, 12) sont prévus, lors de la restitution de l'image traitée, pour visualiser des informations supplémentaires concernant la position spatiale et/ou des attributs déterminés d'objets dans l'image saisie, qui sont associées avec des objets dans l'image saisie, où des informations concernant la distance entre la caméra (7) et des objets dans l'image saisie lors de la restitution de l'image traitée sont associées avec les objets, **caractérisé en ce que** le système d'assistance à la vision comprend en outre un dispositif de stimulation (6) implantable dans le corps sur l'organe de la vision (5) pour la restitution de l'image traitée par stimulation correspondante de l'organe de la vision (5),
où les moyens électroniques (11, 12) sont prévus pour permettre une représentation sélective en profondeur de l'image saisie.

2. Système d'assistance à la vision selon l'une des revendications précédentes, où les informations supplémentaires concernant la position d'objets dans l'image saisie sont indiquées lors de la restitution de l'image traitée par la mise en relief visuelle d'objets déterminés, de préférence par l'utilisation de nuances de gris différentes et/ou de couleurs déterminées pour la visualisation d'objets déterminés.

3. Système d'assistance à la vision selon l'une des revendications précédentes, où des objets dans l'image saisie sont **caractérisés par** une visualisation particulière lors de la restitution de l'image traitée par la visualisation d'informations pour l'éloignement des objets concernés par rapport à la position de l'utilisateur du système d'assistance à la vision (1).

4. Système d'assistance à la vision selon l'une des revendications précédentes, où, par rapport à la position de l'utilisateur du système d'assistance à la vision (1), des objets de plus faible éloignement dans l'image saisie sont **caractérisés par** la visualisation avec des nuances de gris différentes et/ou des couleurs différentes par rapport à des objets de plus grand éloignement lors de la restitution de l'image traitée.

5. Système d'assistance à la vision selon l'une des revendications précédentes, où, par rapport à la position de l'utilisateur du système d'assistance à la vision (1), des objets plus éloignés dans l'image saisie sont masqués lors de la restitution de l'image traitée.

6. Système d'assistance à la vision selon l'une des revendications précédentes, où à chaque point d'image de l'image traitée est associé un vecteur point d'image [Pᵢ = (xᵢ, yᵢ, zᵢ, Eᵢ)] qui comprend des coordonnées spatiales tridimensionnelles [xᵢ, yᵢ, zᵢ] ainsi qu'une variable de propriétés Eᵢ qui représente des attributs déterminés du point d'image concerné.

7. Système d'assistance à la vision selon l'une des revendications précédentes, où une restitution tridimensionnelle de l'image saisie contient des points de quadrillage [Pᵢ = (xᵢ, yᵢ, zᵢ)] qui correspondent à des points d'image déterminés de l'image saisie et un réseau de quadrillage est tendu par des lignes entre des points de quadrillage voisins.

8. Système d'assistance à la vision selon l'une des revendications précédentes, où, dans un mode de fonctionnement déterminé, seuls les objets dans l'image saisie qui se trouvent dans un domaine de saisie déterminé du système d'assistance à la vision (1) sont visualisés lors de la restitution de l'image traitée.

9. Système d'assistance à la vision selon l'une des revendications précédentes, où, sur le système d'assistance à la vision (1) différents modes de fonctionnement peuvent être mis en place, selon lesquels le système d'assistance à la vision (1) traite l'image saisie et restitue l'image traitée, et où le choix des modes de fonctionnement a lieu par le biais d'éléments de commande sur le système d'assistance à la vision (1) ou par le biais d'une télécommande.

10. Système d'assistance à la vision selon la revendication précédente, où, dans un mode de fonctionnement déterminé, le domaine de saisie optique du système d'assistance à la visions (1) est élargi de manière répétée puis de nouveau réduit, de sorte que des images en coupe spatiales sont visualisées en continu avec un domaine de saisie différent.

11. Système d'assistance à la vision selon l'une des revendications précédentes, où à l'aide des moyens électroniques (11, 12) au moyen des données d'image saisies une représentation en perspective à vol d'oiseau ou d'autres perspectives ou échelons de zoom de l'image saisie et des objets qui s'y trouvent est élaborée et visualisée.

12. Système d'assistance à la vision selon l'une des revendications précédentes, où à l'aide des moyens électroniques (11, 12) au moyen des données d'image saisies une esquisse de l'image saisie et des objets qui s'y trouvent est élaborée et visualisée.

13. Système d'assistance à la vision selon l'une des revendications précédentes, où à l'aide des moyens électroniques (11, 12) au moyen des données d'image saisies une restitution tridimensionnelle de l'image saisie et des objets qui s'y trouvent est élaborée et visualisée.

14. Système d'assistance à la vision selon l'une des revendications précédentes, qui comprend en outre une mémoire dans laquelle une bibliothèque d'images avec un certain nombre de motifs d'image peut être mémorisée, et où les moyens électroniques (11, 12) sont agencés pour réaliser une reconnaissance de motifs d'image où les objets dans l'image saisie sont comparés avec des motifs d'image prédéfinis et affectés de manière correspondante.

15. Système d'assistance à la vision selon la revendication précédente, dans lequel l'élaboration d'une restitution tridimensionnelle saisit au moins deux images de positions de la caméra (7) spatialement différentes et traite par les moyens électroniques (11, 12) en tenant compte des positions spatialement différentes et de l'orientation angulaire relative de la caméra (7).

16. Système d'assistance à la vision selon l'une des revendications précédentes, où la caméra (7) est couplée avec au moins un capteur d'accélération (9) qui détermine l'accélération en translation [*x y z*] de la caméra (7) dans les trois directions de l'espace [*x, y, z*] et peut aussi déterminer en fonction du nombre et de la disposition des capteurs d'accélération (9) les accélérations angulaires [*ẍ ÿ z̈* ϕ̈ₓ ϕ̈*_{y}* ϕ̈*_{z}*] de la caméra (7) autour des trois axes de l'espace [*x*, *y*, *z*].

17. Système d'assistance à la vision selon l'une des revendications précédentes, où la caméra (7) est couplée avec au moins un capteur d'accélération angulaire (10) qui détermine l'accélération angulaire [*ẍ ÿ z̈* ϕ̈*ₓ* ϕ̈*_{y}* ϕ̈*_{z}*] de la caméra (7) autour des trois axes de l'espace [*x, y, z*].

18. Système d'assistance à la vision selon l'une des revendications précédentes, où à l'aide des moyens électroniques (11, 12) à l'aide des valeurs mesurées d'accélération angulaire délivrées par un certain nombre de capteurs d'accélération angulaire (10) une modification de l'orientation spatiale de la caméra (7) par rapport à une orientation spatiale précédente de la caméra (7) est déterminée ; et/ou
où le système d'assistance à la vision (1) comprend des intégrateurs électroniques (11, 12) pour l'intégration des valeurs mesurées d'accélération délivrées par un certain nombre de capteurs d'accélération (9, 10) et pour le calcul de la position spatiale et de l'orientation spatiale de la caméra (7).

19. Système d'assistance à la vision selon l'une des revendications précédentes, où une succession d'au moins deux images pour une position de la caméra (7) à chaque fois différente est saisie, de préférence à des intervalles de temps fixes.

20. Système d'assistance à la vision selon l'une des revendications précédentes, où à partir de la succession des images saisies à partir de positions différentes de la caméra à l'aide des moyens électroniques (11, 12) une restitution tridimensionnelle ou stéréoscopique des images saisies et des objets qui s'y trouvent est calculée et visualisée ; et/ou
où à partir de la succession des images saisies à partir de positions différentes de la caméra à l'aide des moyens électroniques (11, 12) la position spatiale et/ou l'orientation spatiale de la caméra (7) sont calculées.

21. Système d'assistance à la vision selon l'une des revendications précédentes, où la au moins une caméra (7) est intégrée dans des lunettes (2) qui peuvent être portées par l'utilisateur comme des lunettes normales.

22. Système d'assistance à la vision selon l'une des revendications précédentes, où il est prévu une deuxième caméra (7) qui saisit des images stéréoscopiques conjointement avec la première caméra (7), où de préférence la deuxième caméra (7) est intégrée dans les lunettes (2) à une position dans les lunettes (2) éloignée de la première caméra (7).

23. Système d'assistance à la vision selon l'une des revendications précédentes, où une vitesse actuelle de la caméra (7) est déterminée par le biais de la modification des données d'image saisies en tenant compte des valeurs mesurées d'accélération déterminées par les capteurs d'accélération (9, 10) couplés avec la caméra (7).

24. Système d'assistance à la vision selon l'une des revendications précédentes, où l'orientation angulaire de la caméra (7) est déterminée par l'évaluation de valeurs de position angulaire précédentes, par un compas, une sonde de Hall et/ou un capteur d'inclinaison basé sur la gravitation.

25. Système d'assistance à la vision selon l'une des revendications précédentes, où il est prévu au moins un capteur de distance pour déterminer l'éloignement d'objets et/ou d'obstacles dans l'image saisie, et où le capteur de distance est conçu de préférence sous forme de capteur à ultrasons ou de scanneur laser rotatif dans le domaine de longueurs d'onde visible ou invisible.

26. Système d'assistance à la vision selon la revendication précédente, où le système d'assistance à la vision (1) comprend une source lumineuse pour éclairer le domaine d'image à saisir par la caméra (7) avec de la lumière (18) dans le domaine de longueurs d'onde visible ou invisible, avec de la lumière infrarouge ou avec de la lumière laser (18), et la caméra (7) du système d'assistance à la visions (1) est sensible dans le domaine de longueurs d'onde correspondant.

27. Système d'assistance à la vision selon la revendication précédente, où le système d'assistance à la vision projette sur le domaine d'image à saisir par la caméra (7) de la lumière (18) avec un motif en bandes homogène ou structuré (16), et où à l'aide des moyens électroniques (11, 12) une restitution tridimensionnelle de l'image saisie et des objets qui s'y trouvent est élaborée et visualisée à partir de distorsions, déformations, déplacements ou d'autres modifications (17) du motif en bandes (16) dans l'image saisie.

28. Système d'assistance à la vision selon l'une des revendications précédentes, où le système d'assistance à la vision pour l'éclairement du domaine d'image à saisir par la caméra (7) avec un motif en bandes (16) comprend un projecteur laser avec une unité de balayage, un projecteur de quadrillage (15) ou un masque de projection qui est positionné dans la trajectoire des rayons d'une source lumineuse.

29. Système d'assistance à la vision selon l'une des revendications précédentes, où le système d'assistance à la vision produit avec la lumière laser (18) des effets d'interférences et projette une figure d'interférences (16) sur le domaine d'image à saisir par la caméra (7).

30. Système d'assistance à la vision selon l'une des revendications précédentes, où le système d'assistance à la vision saisit tour à tour une image du domaine d'image à saisir sous éclairement avec un motif en bandes (16) et une image du domaine d'image à saisir sans éclairement et combine et compare entre elles les données d'image des images saisies à l'aide des moyens électroniques (11, 12).

31. Système d'assistance à la vision selon l'une des revendications précédentes, où le domaine d'image à saisir est exploré au moyen d'un faisceau laser pour obtenir des données d'image et/ou des informations concernant l'éloignement d'objets dans l'image saisie.
